# EUROPEAN PATENT APPLICATION

(11) **EP 4 721 668 A2**
(43) Date of publication of application: **08.04.2026**
(21) Application number: 25206872.1
(22) Date of filing: 06.10.2025
(51) Int. Cl.: A61B 6/00, A61B 6/58

(54) **MOBILE RADIATION GENERATION APPARATUS, METHOD OF OPERATING MOBILE RADIATION GENERATION APPARATUS, AND OPERATION PROGRAM FOR MOBILE RADIATION GENERATION APPARATUS**

(30) Priority: 07.10.2024 JP 2024176072
(71) Applicant: FUJIFILM Corporation, Tokyo 106-8620 (JP)
(72) Inventor: HORIUCHI, Hisatsugu, Tokyo (JP); SHIMIZUKAWA, Sho, Tokyo (JP); KOBAYASHI, Takeyasu, Tokyo (JP); KITAMURA, Moeka, Tokyo (JP)
(74) Representative: Dehns Germany Partnerschaft mbB

(57) **Abstract**

There are provided a mobile radiation generation apparatus (10) that can support registration between a radiation generation unit (15) and a radiation image detector (11) even in a case where the radiation image detector (11) is covered by a subject (H), a method of operating the mobile radiation generation apparatus (10), and an operation program (90) for the mobile radiation generation apparatus (10).

A first angle detection unit (130, 171, 176) of a registration support processing unit (100) detects a first angle (ΨC) about a vertical axis (VA) between a radiation generation unit (15) and a long edge (20) of an examination table (17) to acquire the first angle (ΨC). The first angle (ΨC) corresponds to an angle about a Z axis, which is a normal to a radiation detection surface (22) of an electronic cassette (11), between the radiation generation unit (15) and the electronic cassette (11). A first/second support information output unit (132) of the registration support processing unit (100) outputs first support information (135) for supporting the registration of the radiation generation unit (15) with respect to the angle about the Z axis based on the first angle (ΨC).

## Description

### BACKGROUND OF THE INVENTION

### 1. Field of the Invention

The technology of the present disclosure relates to a mobile radiation generation apparatus, a method of operating the mobile radiation generation apparatus, and an operation program for the mobile radiation generation apparatus.

### 2. Description of the Related Art

A mobile radiation generation apparatus includes a radiation generation unit that includes a radiation tube and a carriage unit that includes wheels, and can be moved by the carriage unit. The mobile radiation generation apparatus is used for so-called bedside imaging in which a medical radiographer images a subject (patient) while visiting hospital rooms. Alternatively, the mobile radiation generation apparatus is also used for imaging in an emergency room. Further, the mobile radiation generation apparatus can also be brought into an operating room and used during surgery. Furthermore, the mobile radiation generation apparatus can also be brought to outdoor disaster sites and used for emergencies.

The mobile radiation generation apparatus is used together with an electronic cassette. The electronic cassette is a radiation image detector in which a sensor panel for detecting a radiation image is built into a portable housing. Before radiography, registration (positioning) between the radiation generation unit and the electronic cassette is performed by an operator such as a medical radiographer.

In order to facilitate the registration between the radiation generation unit and the electronic cassette, JP2023-142507A discloses the following technology. That is, a marker attached to a housing of an electronic cassette (referred to as an X-ray detection unit in JP2023-142507A) is imaged by a camera of a radiation generation unit (referred to as an X-ray irradiation unit in JP2023-142507A). Then, an angle between the radiation generation unit and the electronic cassette is detected based on the position, the angle, the size, and the like of the marker shown in a captured image. The angle includes an angle (referred to as a yaw angle in JP2023-142507A) about a normal to a radiation detection surface of the electronic cassette. Next, a deviation amount between the detected angle and a set angle is calculated. Then, the display form of an irradiation position mark for radiation projected onto the subject is changed depending on the magnitude of the deviation amount. An operator moves the radiation generation unit to reduce a deviation from the set angle, depending on the display of the irradiation position mark.

### SUMMARY OF THE INVENTION

In the technology disclosed in JP2023-142507A, the marker cannot be imaged by the camera in a case where the electronic cassette is covered by the subject. Accordingly, the angle cannot be detected. Therefore, it is also not possible to change the display form of the irradiation position marker according to the detected angle and to support the registration between the radiation generation unit and the electronic cassette.

One embodiment according to the technology of the present disclosure provides a mobile radiation generation apparatus that can support registration between a radiation generation unit and a radiation image detector even in a case where the radiation image detector is covered by a subject, a method of operating the mobile radiation generation apparatus, and an operation program for the mobile radiation generation apparatus.

According to an aspect of the present disclosure, there is provided a mobile radiation generation apparatus that includes a radiation generation unit emitting radiation to a radiation image detector disposed between a subject and an examination table on which the subject lies. The mobile radiation generation apparatus includes a processor. The processor is configured to: acquire a first angle about a vertical axis between the radiation generation unit and an edge of the examination table that corresponds to an angle about a normal to a radiation detection surface of the radiation image detector between the radiation generation unit and the radiation image detector; and output first support information for supporting registration of the radiation generation unit with respect to the angle about the normal based on the first angle.

It is preferable that the mobile radiation generation apparatus further includes a first display unit and the processor is configured to control a display based on the first support information that is shown on the first display unit.

It is preferable that the processor is configured to: output a first deviation amount between the first angle and a first set angle as the first support information; and perform a display, which shows a movement direction and/or a movement amount of the radiation generation unit for reducing the first deviation amount, as the display based on the first support information.

It is preferable that the processor is configured to: acquire a captured image of the examination table; and detect the first angle based on the captured image.

It is preferable that the processor is configured to: extract a contour line of the edge of the examination table from the captured image, and detect an angle at which a difference between the contour line and a pre-registered reference contour line is a minimum value, as the first angle.

It is preferable that the mobile radiation generation apparatus further includes a geomagnetic sensor, and the processor is configured to: acquire an azimuthal angle detection result output from the geomagnetic sensor; and detect a difference between the azimuthal angle detection result and a pre-registered azimuthal angle of the examination table as the first angle.

It is preferable that the mobile radiation generation apparatus further includes a gyro sensor, and the processor is configured to: acquire an angular velocity detection result output from the gyro sensor; and detect a difference between the angular velocity detection result and a pre-registered angle of the examination table as the first angle.

It is preferable that the processor is configured to: acquire even a second angle about an axis, which is parallel to a side of the radiation detection surface, between the radiation generation unit and the radiation image detector; and output second support information for supporting registration of the radiation generation unit with respect to an angle about the axis based on the second angle.

It is preferable that the mobile radiation generation apparatus further includes a second display unit, and the processor is configured to control a display based on the second support information that is shown on the second display unit.

It is preferable that the processor is configured to: output a second deviation amount between the second angle and a second set angle as the second support information; and perform a display, which shows a movement direction and/or a movement amount of the radiation generation unit for reducing the second deviation amount, as the display based on the second support information.

It is preferable that the processor is configured to: acquire a posture detection result output from a posture detection sensor provided in the radiation image detector; and detect the second angle based on the posture detection result.

It is preferable that the mobile radiation generation apparatus further includes a support column and an arm part that is attached to the support column, and the radiation generation unit is disposed at a distal end of the arm part.

It is preferable that the arm part includes a joint point that is used to change an angle of the arm part relative to the support column.

It is preferable that the arm part includes a link mechanism for keeping the radiation generation unit in a horizontal posture.

It is preferable that the mobile radiation generation apparatus further includes a first rotation mechanism that rotates the radiation generation unit about the vertical axis and a second rotation mechanism that rotates the radiation generation unit about a horizontal axis.

It is preferable that the mobile radiation generation apparatus further includes a support column and an arm part that is attached to the support column, the radiation generation unit is disposed at a distal end of the arm part, the arm part is connected to a joint point that is used to change an angle of the arm part relative to the support column, the first rotation mechanism is positioned closer to the radiation generation unit than the joint point, and the second rotation mechanism is positioned closer to the radiation generation unit than the first rotation mechanism.

It is preferable that the processor is configured to: acquire a distance measurement result between a focus of the radiation and the radiation detection surface; and output third support information for supporting adjustment of a distance between the focus of the radiation and the radiation detection surface to a set distance based on the distance measurement result.

It is preferable that the mobile radiation generation apparatus further includes a third display unit and the processor is configured to control a display based on the third support information that is shown on the third display unit.

It is preferable that the processor is configured to: acquire a captured image of the subject; detect an irradiation reference position for the radiation on the subject based on the captured image; and output fourth support information for supporting registration between a focus of the radiation and the irradiation reference position.

It is preferable that the mobile radiation generation apparatus further includes a fourth display unit and the processor is configured to control a display based on the fourth support information that is shown on the fourth display unit.

According to another aspect of the present disclosure, there is provided a method of operating a mobile radiation generation apparatus that includes a radiation generation unit emitting radiation to a radiation image detector disposed between a subject and an examination table on which the subject lies. The method includes: acquiring a first angle about a vertical axis between the radiation generation unit and an edge of the examination table that corresponds to an angle about a normal to a radiation detection surface of the radiation image detector between the radiation generation unit and the radiation image detector; and outputting first support information for supporting registration of the radiation generation unit with respect to the angle about the normal based on the first angle.

According to still another aspect of the present disclosure, there is provided an operation program for a mobile radiation generation apparatus that includes a radiation generation unit emitting radiation to a radiation image detector disposed between a subject and an examination table on which the subject lies. The operation program causes a computer to execute processing comprising: acquiring a first angle about a vertical axis between the radiation generation unit and an edge of the examination table that corresponds to an angle about a normal to a radiation detection surface of the radiation image detector between the radiation generation unit and the radiation image detector; and outputting first support information for supporting registration of the radiation generation unit with respect to the angle about the normal based on the first angle.

According to the technology of the present disclosure, it is possible to provide a mobile radiation generation apparatus that can support registration between a radiation generation unit and a radiation image detector even in a case where the radiation image detector is covered by a subject, a method of operating the mobile radiation generation apparatus, and an operation program for the mobile radiation generation apparatus.

### BRIEF DESCRIPTION OF THE DRAWINGS

FIG. 1 is a diagram showing a manner of imaging using a radiography system.
FIG. 2 is a diagram showing the radiography system.
FIG. 3 is a diagram showing a UI-based device.
FIG. 4 is a diagram showing a joint point and a link mechanism.
FIG. 5 is a diagram showing a first rotation mechanism and a second rotation mechanism.
FIG. 6 is a diagram showing a rotational position detection sensor group and a rotational position detection result group.
FIG. 7 is a diagram showing a camera.
FIG. 8 is a diagram showing an indicator.
FIG. 9 is a block diagram showing an electrical configuration of a mobile radiation generation apparatus.
FIG. 10 is a block diagram showing processing units of a CPU.
FIG. 11 is a diagram showing an irradiation condition table.
FIG. 12 is a diagram showing a semi-sitting position.
FIG. 13 is a diagram showing radiography.
FIG. 14 is a diagram showing a coordinate system related to an electronic cassette, a coordinate system related to a radiation generation unit, and angles about respective axes of the respective coordinate systems.
FIG. 15 is a diagram showing a first angle about a vertical axis between the radiation generation unit and a long edge of an examination table.
FIG. 16 is a diagram showing a detailed configuration of a registration support processing unit.
FIG. 17 is a diagram showing a detailed configuration of a first angle detection unit.
FIG. 18 is a diagram showing processing of a calculation unit.
FIG. 19 is a graph showing the sum of differences between the contour line of an edge of the examination table and a reference contour line with respect to a rotation angle of the reference contour line.
FIG. 20 is a diagram showing a display based on first support information and second support information shown on an indicator.
FIG. 21 is a flowchart showing a procedure of registration support processing of the mobile radiation generation apparatus.
FIG. 22 is a diagram showing a second embodiment in which the first angle is detected based on an azimuthal angle detection result obtained from a geomagnetic sensor.
FIG. 23 is a diagram showing the second embodiment in which the first angle is detected based on the azimuthal angle detection result obtained from the geomagnetic sensor.
FIG. 24 is a diagram showing a third embodiment in which the first angle is detected based on an angular velocity detection result obtained from a gyro sensor.
FIG. 25 is a diagram showing the third embodiment in which the first angle is detected based on the angular velocity detection result obtained from the gyro sensor.
FIG. 26 is a diagram showing a fourth embodiment in which third support information is output based on a distance measurement result obtained from a distance measurement sensor.
FIG. 27 is a diagram showing a display based on the third support information that is shown on the indicator.
FIG. 28 is a diagram showing a fifth embodiment in which a position detection result as fourth support information is output based on a captured image of a subject obtained from a camera.
FIG. 29 is a diagram showing a display based on the fourth support information that is shown on the indicator.

### DESCRIPTION OF THE PREFERRED EMBODIMENTS

### First embodiment

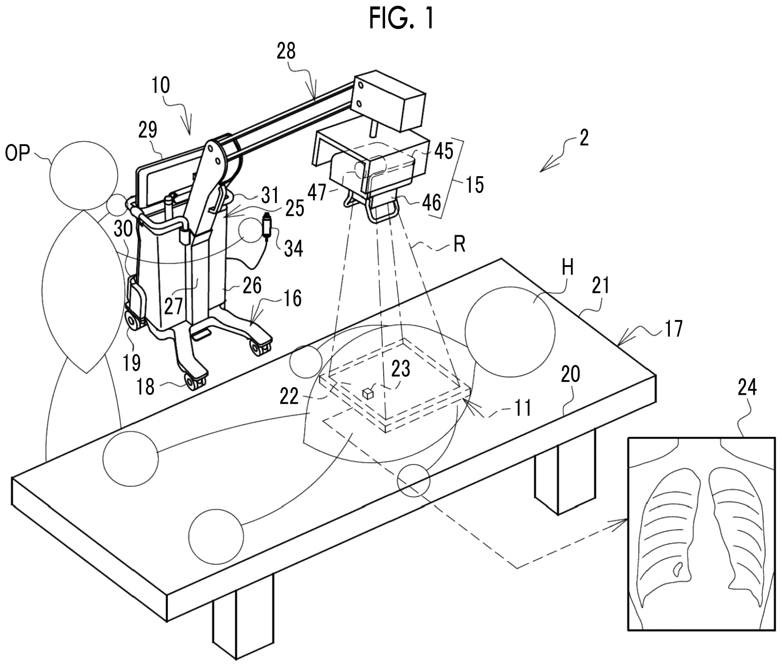

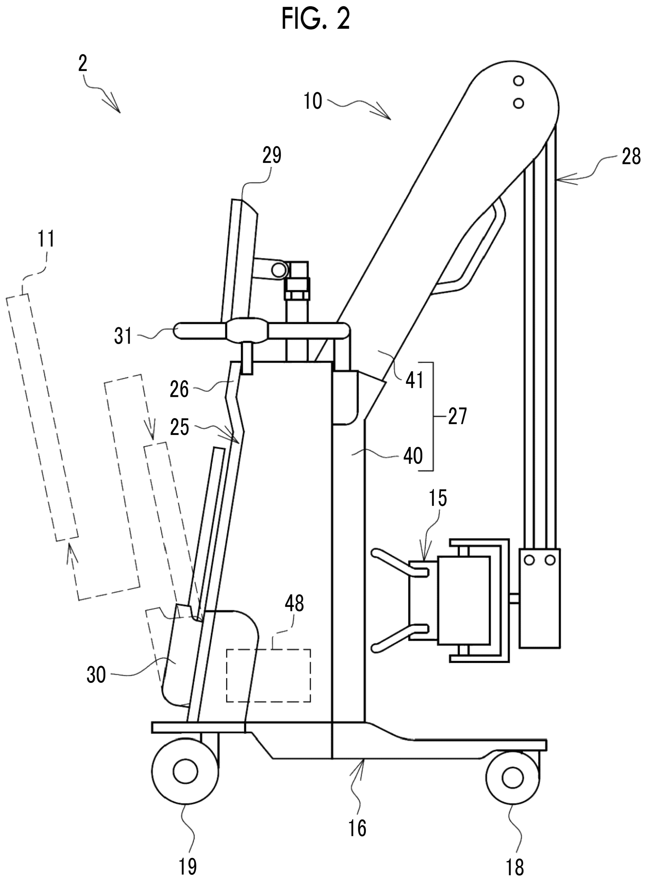

For example, as shown in FIGS. 1 and 2, a radiography system 2 includes a mobile radiation generation apparatus 10 and an electronic cassette 11. The mobile radiation generation apparatus 10 includes a radiation generation unit 15 and a carriage unit 16. The radiation generation unit 15 emits radiation R to, for example, a subject (patient) H lying on an examination table 17. The carriage unit 16 includes a pair of right and left front wheels 18 and a pair of right and left rear wheels 19. The mobile radiation generation apparatus 10 can be moved within a hospital by the carriage unit 16. The mobile radiation generation apparatus 10 is used for so-called bedside imaging in which a medical radiographer images the subject H while visiting hospital rooms. For this reason, the mobile radiation generation apparatus 10 is also referred to as a bedside X-ray machine. Further, the mobile radiation generation apparatus 10 can also be brought into an operating room and used during surgery. Furthermore, the mobile radiation generation apparatus 10 can also be brought to outdoor disaster sites and used for emergencies.

The examination table 17 has a size slightly larger than that of the subject H. The examination table 17 is a bed installed in a hospital room, an operating table installed in an operating room, a stretcher, a futon, or the like. In FIG. 1, a bed is illustrated as the examination table 17. The examination table 17 has a rectangular shape and has long edges 20 extending along a craniocaudal axis of the subject H and short edges 21 extending along a lateral axis of the subject H.

As is well known, in the electronic cassette 11, a sensor panel is built into a portable rectangular housing and is driven wirelessly by a battery. As is also well known, the sensor panel has a configuration in which a plurality of pixels that are sensitive to the radiation R or visible light converted from the radiation R to generate signal charges are arranged. The electronic cassette 11 includes a rectangular radiation detection surface 22 corresponding to the sensor panel. Further, for example, a posture detection sensor 23, such as an acceleration sensor or a gyro sensor, is built into the electronic cassette 11. The electronic cassette 11 is disposed between the subject H and the examination table 17 (below the subject H). For this reason, the electronic cassette 11 may be covered by the subject H. The electronic cassette 11 receives the radiation R emitted from the radiation generation unit 15 and transmitted through the subject H, and outputs a radiation image 24. The electronic cassette 11 is an example of "radiation image detector" according to the technology of the present disclosure.

A body part 25 is mounted on the carriage unit 16. The body part 25 includes a central part 26, a support column part 27, an arm part 28, and the like in addition to the above-described radiation generation unit 15.

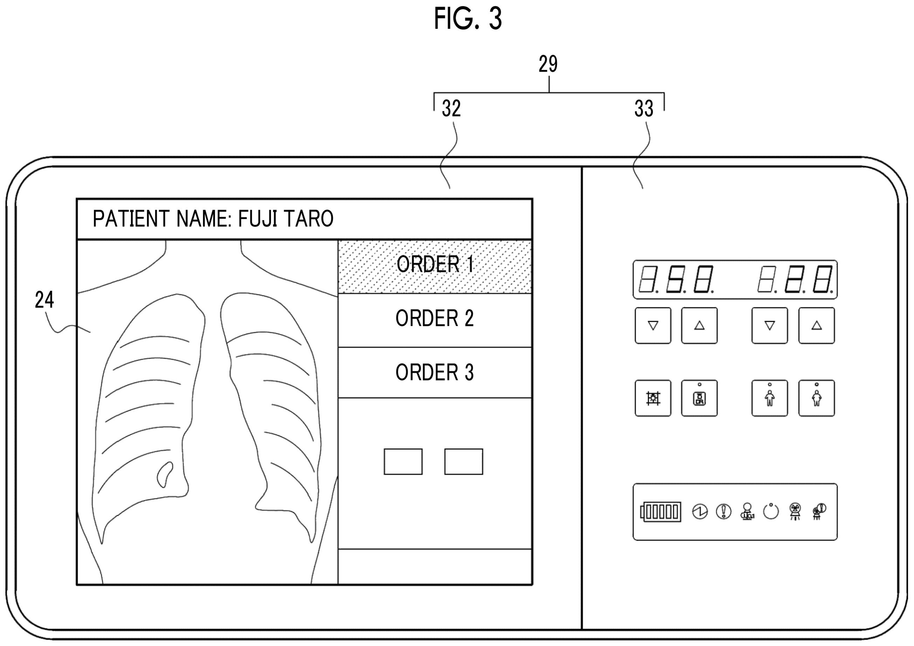

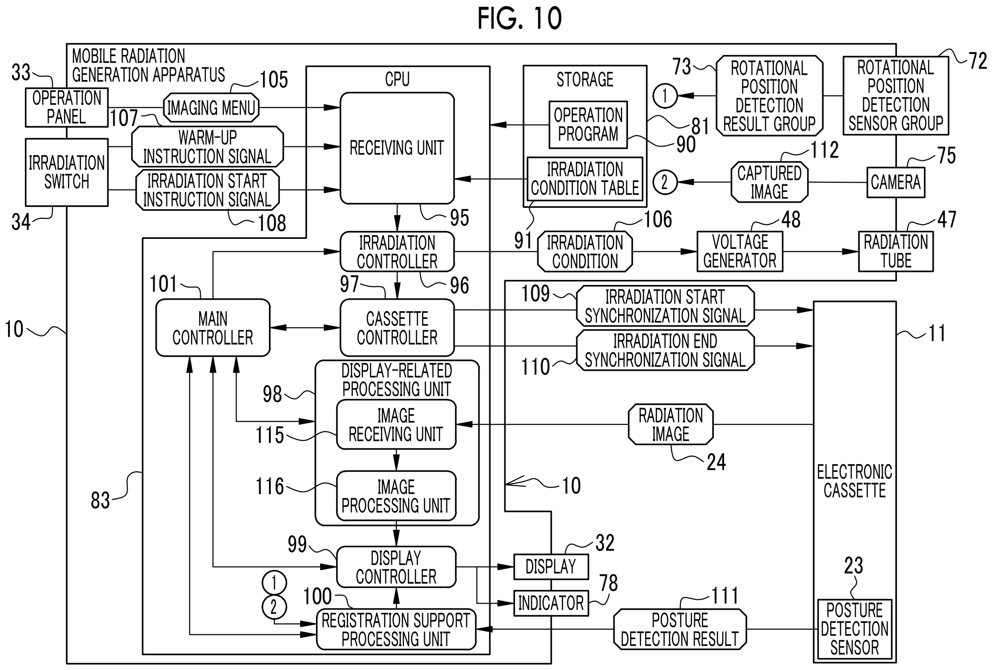

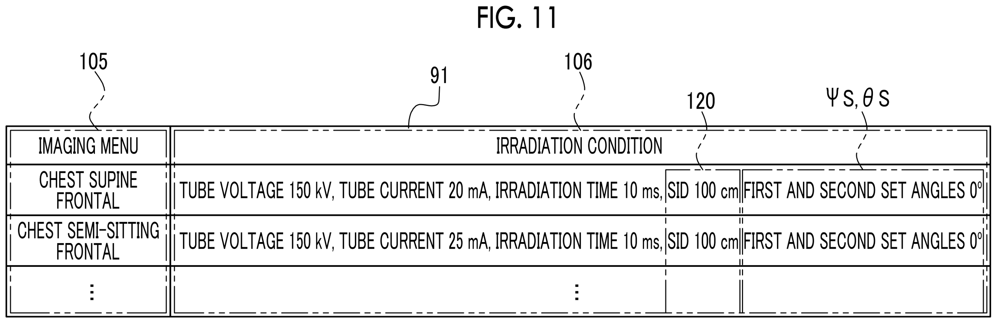

The central part 26 includes a user interface (UI)-based device 29, a cassette storage portion 30, and a handle 31. For example, as shown in FIG. 3, the UI-based device 29 includes a touch panel display (hereinafter, simply referred to as a display) 32 and an operation panel 33. The display 32 displays the radiation image 24 and the like. The operation panel 33 is operated by an operator OP, such as a medical radiographer, in setting irradiation conditions 106 (see FIGS. 10 and 11) of the radiation R.

The cassette storage portion 30 is provided on a rear portion side of the central part 26. The cassette storage portion 30 stores the electronic cassette 11. There are a plurality of types of electronic cassettes 11 having vertical and horizontal sizes, such as 17 inches × 17 inches, 17 inches × 14 inches, and 12 inches × 10 inches. The cassette storage portion 30 can store such a plurality of types of electronic cassettes 11 regardless of the types. Further, the cassette storage portion 30 has a function of charging a battery of the stored electronic cassette 11.

The handle 31 is provided to surround an upper portion of the central part 26. The operator OP grips the handle 31 to operate the carriage unit 16, and by extension, the mobile radiation generation apparatus 10. The operator OP moves the mobile radiation generation apparatus 10 while gripping the handle 31 in a state shown in FIG. 2 in which the radiation generation unit 15 is stored above the carriage unit 16 and in front of the central part 26.

An irradiation switch 34 is attached to the central part 26. The irradiation switch 34 is a switch that allows the operator OP to give an instruction to start the irradiation with radiation. Since an extension cable is connected to the irradiation switch 34, the irradiation switch 34 can be detached from the central part 26 for use. The irradiation switch 34 is, for example, a two-stage push-button switch. The irradiation switch 34 generates a warm-up instruction signal 107 (see FIG. 10) when being pushed to the first stage (half-pushed), and generates an irradiation start instruction signal 108 (see FIG. 10) when being pushed to the second stage (fully pushed).

The support column part 27 has the shape of a polygonal prism, and stands upright at the center of the carriage unit 16. The support column part 27 includes a first support column 40 that stands upright on the upper surface of the carriage unit 16 and a second support column 41 that extends continuously upward from the first support column 40 at a predetermined angle. The first support column 40 and the second support column 41 are an example of "support column" according to the technology of the present disclosure.

A proximal end of the arm part 28 is attached to the support column part 27, more specifically, the second support column 41, and the radiation generation unit 15 is disposed at a distal end of the arm part 28 that is a free end opposite to the proximal end. As will be described in detail later, the arm part 28 can be folded relative to the second support column 41. Further, as will be described in detail later, the radiation generation unit 15 can be rotated or swung left and right relative to the arm part 28.

The radiation generation unit 15 includes a radiation source 45 and an irradiation field limiter 46. A radiation tube 47 is built into the radiation source 45. The radiation tube 47 generates, for example, X-rays as the radiation R. A filament, a target, a grid electrode, and the like (none of which are shown) are provided in the radiation tube 47. A voltage is applied between the filament as a cathode and the target as an anode from a voltage generator 48 built into the central part 26. The voltage applied between the filament and the target is referred to as a tube voltage. The filament releases thermal electrons, which correspond to the applied tube voltage, toward the target. The target emits the radiation R when the thermal electrons released from the filament collide with the target. The grid electrode is disposed between the filament and the target. The grid electrode changes a flow rate of the thermal electrons from the filament toward the target according to the voltage applied from the voltage generator 48. The flow rate of the thermal electrons from the filament toward the target is referred to as a tube current. The tube voltage and the tube current are set as irradiation conditions 106 along with an irradiation time.

In a case where the irradiation switch 34 is half-pushed and the warm-up instruction signal 107 is generated, the filament is warmed up and the rotation of the target is started. When the filament reaches a prescribed temperature and the target reaches a prescribed rotation speed, warm-up is completed. In a case where the irradiation switch 34 is fully pushed and the irradiation start instruction signal 108 is generated in a state in which the warm-up is completed, the tube voltage is applied from the voltage generator 48 and the radiation R is generated from the radiation tube 47. When the irradiation time set in the irradiation conditions 106 has elapsed from the start of generation of the radiation R, the application of the tube voltage is stopped and the irradiation with the radiation Rends.

The irradiation field limiter 46 limits an irradiation field of the radiation R generated from the radiation tube 47. For example, the irradiation field limiter 46 has a configuration in which four shield plates made of lead or the like blocking the radiation R are disposed on respective sides of a quadrangle and a quadrangular emission opening transmitting radiation is formed at a central portion. The irradiation field limiter 46 changes the position of each shield plate to change the size of the emission opening. As a result, the irradiation field limiter 46 changes the irradiation field of the radiation R.

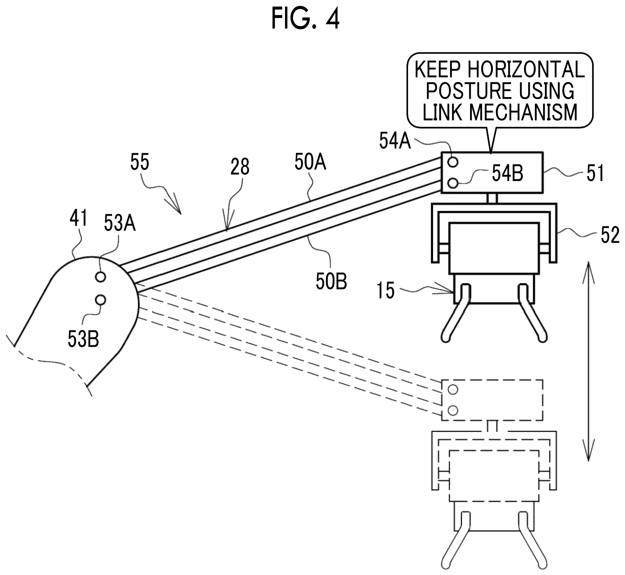

For example, as shown in FIG. 4, the arm part 28 includes two parallel arms 50A and 50B, a first connecting member 51, and a second connecting member 52. The first connecting member 51 has a rectangular parallelepiped shape and connects the arms 50A and 50B to the second connecting member 52. The second connecting member 52 has a U-shaped cross section and connects the first connecting member 51 and the radiation generation unit 15. That is, the arms 50A and 50B are connected to the radiation generation unit 15 via the first connecting member 51 and the second connecting member 52. The term "parallel" mentioned herein refers to not only "completely parallel" but also "parallel" in the sense of including an error that is generally allowed in a technical field to which the technology of the present disclosure belongs.

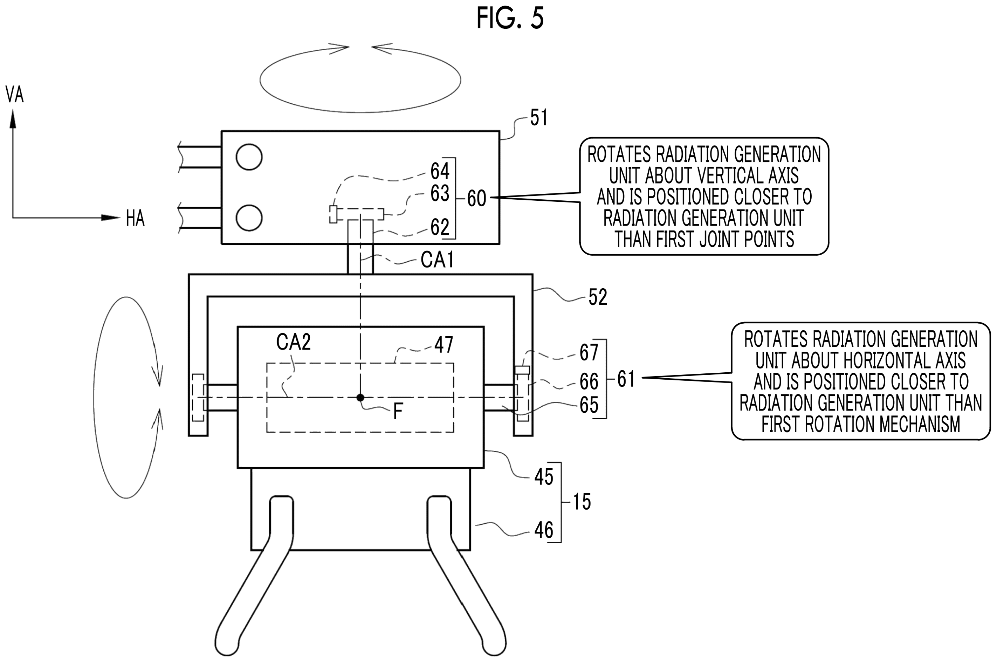

Proximal ends of the arms 50A and 50B are connected to a distal end of the second support column 41 via first joint points 53A and 53B, respectively. Further, distal ends of the arms 50A and 50B are connected to the first connecting member 51 via second joint points 54A and 54B, respectively. The arms 50A and 50B can be folded relative to the second support column 41 with the first joint points 53A and 53B as fulcrums. As the arms 50A and 50B are folded relative to the second support column 41 in this way, the radiation generation unit 15 is moved up and down along a vertical axis VA (see FIG. 5). The first joint points 53A and 53B are an example of "joint point" according to the technology of the present disclosure.

The arms 50A and 50B, the first joint points 53A and 53B, and the second joint points 54A and 54B constitute a link mechanism 55. The link mechanism 55 keeps the first connecting member 51, and by extension, the radiation generation unit 15 in a horizontal posture. For this reason, even in a case where the arms 50A and 50B are folded relative to the second support column 41 and the radiation generation unit 15 is moved up and down, the posture of the radiation generation unit 15 is kept horizontal. The term "horizontal" mentioned herein refers to not only "completely horizontal" but also "horizontal" in the sense of including an error that is generally allowed in a technical field to which the technology of the present disclosure belongs.

For example, as shown in FIG. 5, the first connecting member 51 is provided with a first rotation mechanism 60 and the second connecting member 52 is provided with a second rotation mechanism 61. For this reason, the first rotation mechanism 60 is positioned closer to the radiation generation unit 15 than the first joint points 53A and 53B. Further, the second rotation mechanism 61 is positioned closer to the radiation generation unit 15 than the first rotation mechanism 60. The first rotation mechanism 60 includes a first rotating shaft 62, a first bearing 63, and a first rotational position detection sensor 64. Similarly, the second rotation mechanism 61 includes a second rotating shaft 65, a second bearing 66, and a second rotational position detection sensor 67.

The first rotating shaft 62 is one cylindrical rod parallel to the vertical axis VA, and a central axis CA1 thereof coincides with a focus F of the radiation R generated from the radiation tube 47. One end of the first rotating shaft 62 is connected to the second connecting member 52, and the other end thereof is connected to the first bearing 63. The first rotating shaft 62 rotates relative to the first bearing 63. For this reason, the second connecting member 52, and by extension, the radiation generation unit 15 can be rotated about the vertical axis VA by the first rotation mechanism 60.

The second rotating shaft 65 is formed of a pair of front and rear cylindrical rods that are parallel to a horizontal axis HA extending to the front and rear, and a central axis CA2 thereof coincides with the focus F. One end of the second rotating shaft 65 is connected to the radiation generation unit 15 (radiation source 45), and the other end thereof is connected to the second bearing 66. The second rotating shaft 65 rotates relative to the second bearing 66. For this reason, the radiation generation unit 15 can be rotated (swung left and right) about the horizontal axis HA by the second rotation mechanism 61. The term "coincide" mentioned herein refers to not only "completely coincide" but also "coincide" in the sense of including an error that is generally allowed in a technical field to which the technology of the present disclosure belongs.

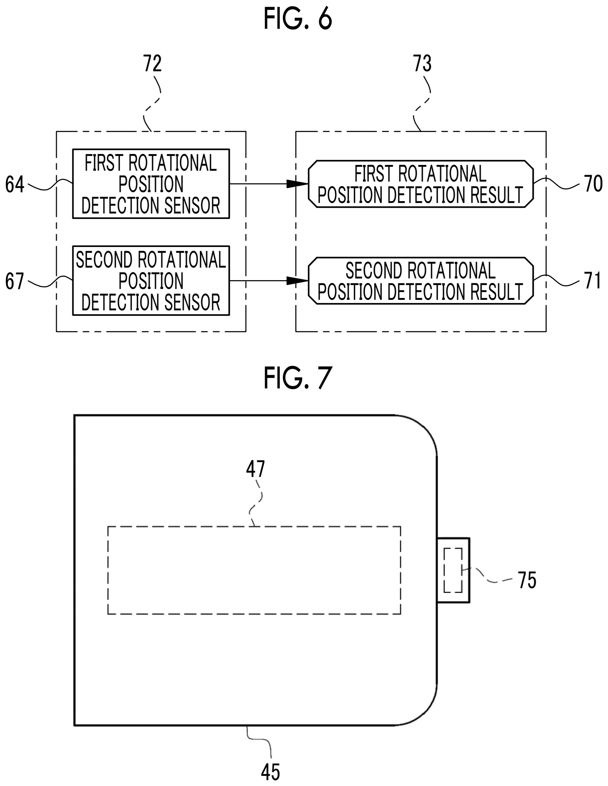

The first rotational position detection sensor 64 and the second rotational position detection sensor 67 are, for example, rotary encoders or potentiometers. The first rotational position detection sensor 64 detects the rotational position of the first rotating shaft 62 relative to the first bearing 63, and by extension, the rotational position of the radiation generation unit 15 about the vertical axis VA. Similarly, the second rotational position detection sensor 67 detects the rotational position of the second rotating shaft 65 relative to the second bearing 66, and by extension, the rotational position of the radiation generation unit 15 about the horizontal axis HA. For example, as shown in FIG. 6, the first rotational position detection sensor 64 outputs a first rotational position detection result 70 that is a detection result of the rotational position of the radiation generation unit 15 about the vertical axis VA. Further, the second rotational position detection sensor 67 outputs a second rotational position detection result 71 that is a detection result of the rotational position of the radiation generation unit 15 about the horizontal axis HA. In the following description, the first rotational position detection sensor 64 and the second rotational position detection sensor 67 will be collectively referred to as a rotational position detection sensor group 72. Furthermore, the first rotational position detection result 70 and the second rotational position detection result 71 will be collectively referred to as a rotational position detection result group 73.

For example, as shown in FIG. 7, a camera 75 is attached to one side surface of the radiation source 45. The camera 75 images the subject H, the examination table 17, and the like positioned below the radiation source 45 at a predetermined frame rate such as 10 frames/second.

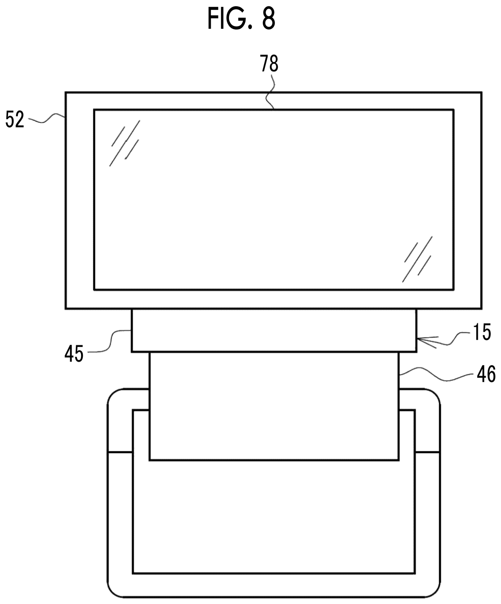

For example, as shown in FIG. 8, an indicator 78 is provided on the rear surface of the second connecting member 52. The indicator 78 is, for example, an organic electroluminescence (EL) panel, and displays various types of information that contribute to registration between the radiation generation unit 15 and the electronic cassette 11. The indicator 78 is an example of "first display unit", "second display unit", "third display unit", and "fourth display unit" according to the technology of the present disclosure.

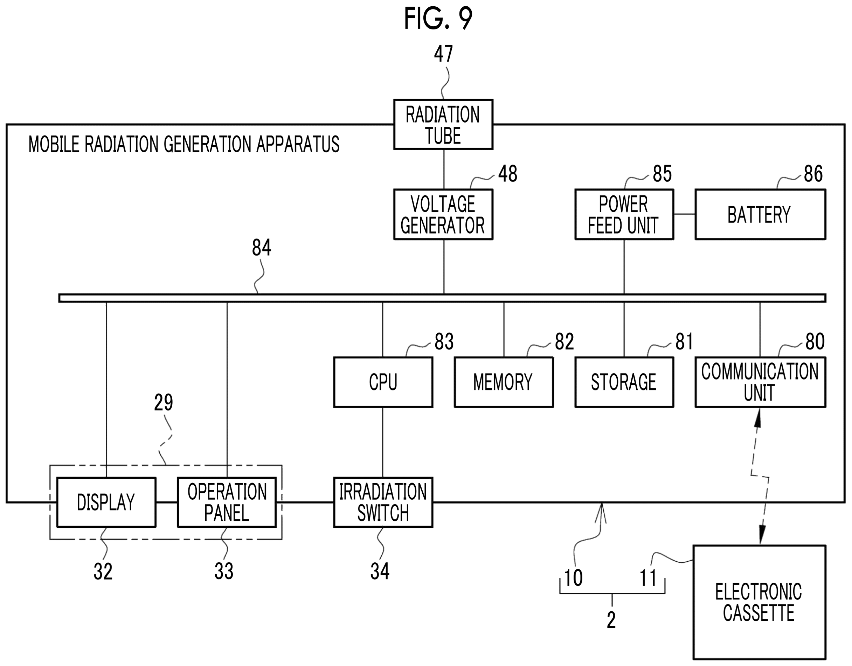

For example, as shown in FIG. 9, the mobile radiation generation apparatus 10 includes a communication unit 80, a storage 81, a memory 82, and a central processing unit (CPU) 83. The communication unit 80, the storage 81, the memory 82, and the CPU 83 are connected via a busline 84 to be capable of communicating with each other. The UI-based device 29 and the voltage generator 48 are also connected to the busline 84. The communication unit 80, the storage 81, the memory 82, the CPU 83, the busline 84, and the UI-based device 29 are an example of "computer" according to the technology of the present disclosure.

The communication unit 80 includes a wireless communication interface that wirelessly communicates with the electronic cassette 11. The communication unit 80 includes a network interface that wirelessly communicates with an external device other than the electronic cassette 11 via a network. Examples of the external device include a radiology information system (RIS) that manages information such as an imaging order, and a picture archiving and communication systems (PACS) that stores the radiation image 24. Further, examples of the network include a wide area network (WAN), such as the Internet or a public communication network.

The storage 81 is, for example, a hard disk drive, a solid state drive, or the like, and stores various programs and various types of data associated with the various programs. The memory 82 is a work memory that is used in a case where the CPU 83 executes processing. The CPU 83 reads out a program stored in the storage 81 to the memory 82 and executes processing according to the read-out program. Accordingly, the CPU 83 collectively controls the operations of the respective units of the mobile radiation generation apparatus 10. The CPU 83 is an example of "processor" according to the technology of the present disclosure.

The above-described irradiation switch 34 is connected to the CPU 83. The irradiation switch 34 outputs the warm-up instruction signal 107 and the irradiation start instruction signal 108 to the CPU 83.

A power feed unit 85 is connected to the busline 84. The power feed unit 85 supplies power from a battery 86 to each unit of the mobile radiation generation apparatus 10. The power feed unit 85 includes a direct-current (DC)-DC converter that converts a direct-current voltage from the battery 86 into a voltage having a value corresponding to a supply destination, a voltage stabilization circuit that stabilizes the value of the converted voltage, and the like. The battery 86 is built into, for example, the central part 26. In this way, the mobile radiation generation apparatus 10 is driven by the battery 86 in a wireless manner. In a case where a plug (not shown) of a power cord, which extends from a lower portion of the body part 25, is connected to a socket of a commercial power supply, the battery 86 is charged or the mobile radiation generation apparatus 10 can be operated with electric power from the commercial power supply.

For example, as shown in FIG. 10, an operation program 90 is stored in the storage 81. The operation program 90 is a program allowing the computer, which includes the UI-based device 29, the storage 81, the memory 82, the CPU 83, and the busline 84, to operate as "mobile radiation generation apparatus" according to the technology of the present disclosure. That is, the operation program 90 is an example of "an operation program for a mobile radiation generation apparatus" according to the technology of the present disclosure. An irradiation condition table 91 is also stored in the storage 81.

The CPU 83 executes the operation program 90 to function as a receiving unit 95, an irradiation controller 96, a cassette controller 97, a display-related processing unit 98, a display controller 99, a registration support processing unit 100, and a main controller 101 in cooperation with the memory 82 and the like.

The receiving unit 95 receives an imaging menu 105 that is input from the operator OP via the operation panel 33. The receiving unit 95 reads out the irradiation conditions 106 corresponding to the received imaging menu 105 from the irradiation condition table 91 and outputs the read-out irradiation conditions 106 to the irradiation controller 96.

The receiving unit 95 also receives the warm-up instruction signal 107 and the irradiation start instruction signal 108 output from the irradiation switch 34. The receiving unit 95 outputs the reception of the warm-up instruction signal 107 and the reception of the irradiation start instruction signal 108 to the irradiation controller 96.

The irradiation controller 96 controls the operation of the radiation tube 47 to control the irradiation with the radiation R. The irradiation controller 96 sets the irradiation conditions 106 in the voltage generator 48. The irradiation controller 96 causes the radiation tube 47 to perform warm-up in a case where the reception of the warm-up instruction signal 107 is input from the receiving unit 95. Further, the irradiation controller 96 causes the radiation R to be emitted from the radiation tube 47 via the voltage generator 48 under the set irradiation conditions 106 in a case where the reception of the irradiation start instruction signal 108 is input from the receiving unit 95.

The irradiation controller 96 outputs the start of the irradiation with the radiation R to the cassette controller 97 in conformity with an irradiation start timing for the radiation R. Further, the irradiation controller 96 outputs the end of the irradiation with the radiation R to the cassette controller 97 in conformity with an irradiation end timing for the radiation R.

The cassette controller 97 transmits various control signals to the electronic cassette 11 via the communication unit 80 to control the operation of the electronic cassette 11. The cassette controller 97 transmits an irradiation start synchronization signal 109 to the electronic cassette 11 in a case where the start of the irradiation with the radiation R is input from the irradiation controller 96. Further, the cassette controller 97 transmits an irradiation end synchronization signal 110 to the electronic cassette 11 in a case where the end of the irradiation with the radiation R is input from the irradiation controller 96. Although not shown, the cassette controller 97 transmits gain values and the like of the signal charges corresponding to the irradiation conditions 106 to the electronic cassette 11.

The display-related processing unit 98 executes display-related processing for displaying the radiation image 24 at an imaging site to allow the operator OP to check the appearance of the radiation image 24. The display-related processing unit 98 includes an image receiving unit 115 and an image processing unit 116. The image receiving unit 115 performs receiving processing for receiving the radiation image 24 from the electronic cassette 11 via the communication unit 80. The image receiving unit 115 outputs the received radiation image 24 to the image processing unit 116.

The image processing unit 116 performs image processing for processing the radiation image 24 for display. Specifically, the image processing unit 116 performs offset correction processing, sensitivity correction processing, defective pixel correction processing, and the like as the image processing. The offset correction processing is processing for subtracting an offset correction image, which is detected in a state in which there is no irradiation with the radiation R, from the radiation image 24 in units of pixels. The image processing unit 116 performs the offset correction processing to remove fixed pattern noise, which is caused by dark charges or the like, from the radiation image 24. The sensitivity correction processing is processing for correcting a variation in the sensitivity of each pixel, a variation in the output characteristics of a circuit that reads out the signal charges, and the like based on sensitivity correction data. The defective pixel correction processing is processing for linearly interpolating a pixel value of a defective pixel with pixel values of surrounding normal pixels, based on information on a defective pixel having an abnormal pixel value that is generated during shipment, periodic inspection, or the like. The image processing unit 116 outputs the radiation image 24, which has been subjected to various types of image processing, to the display controller 99.

As shown in FIG. 3, the display controller 99 performs control to display the radiation image 24 on the display 32. Further, the display controller 99 also controls the display of various types of information that is shown on the indicator 78.

The rotational position detection result group 73 output from the rotational position detection sensor group 72, a posture detection result 111 output from the posture detection sensor 23 of the electronic cassette 11, and a captured image 112 obtained from the camera 75 are input to the registration support processing unit 100. The registration support processing unit 100 performs registration support processing for supporting registration between the radiation generation unit 15 and the electronic cassette 11 based on the rotational position detection result group 73, the posture detection result 111, and the captured image 112.

The main controller 101 collectively controls the operations of the irradiation controller 96, the cassette controller 97, the display-related processing unit 98, the display controller 99, and the registration support processing unit 100.

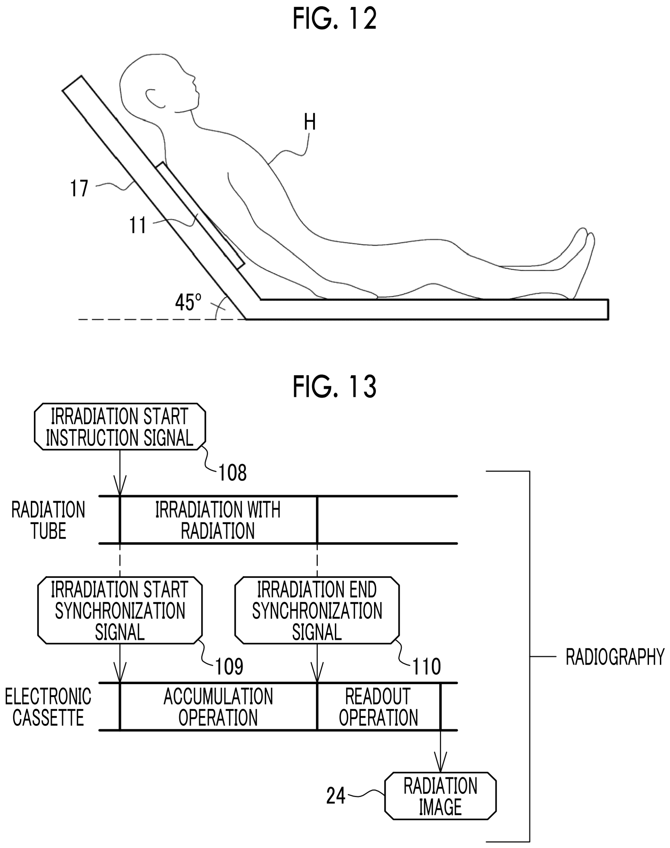

For example, as shown in FIG. 11, the irradiation conditions 106 corresponding to various imaging menus 105 are registered in the irradiation condition table 91. The imaging menus 105 define imaging procedures, such as "chest supine frontal" and "chest semi-sitting frontal," where an imaging region, a posture, and an imaging direction form a set. Examples of the imaging region include a head, a neck, an abdomen, a waist, a shoulder, an elbow, a hand, a knee, and an ankle, in addition to the chest. Examples of the posture include a standing position and a sitting position, in addition to a supine position and a semi-sitting position. Examples of the imaging direction include a back surface and a side surface, in addition to the front surface. Incidentally, the semi-sitting position is a posture in which the upper body is raised by, for example, about 45° while the lower limbs of the subject H lying on the examination table 17 are kept horizontal as shown in, for example, FIG. 12, and is also referred to as a Fowler position. This semi-sitting position allows the subject H to breathe more easily, and can reduce the compression of the lungs by the abdominal organs.

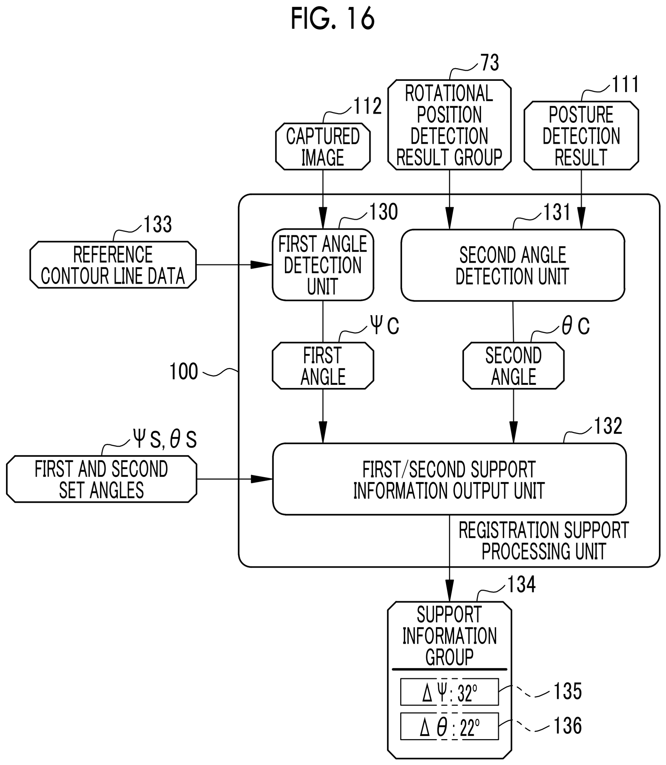

As described above, the irradiation conditions 106 are a set of a tube voltage, a tube current, and an irradiation time. Further, the irradiation conditions 106 also include a source to image receptor distance (SID) 120, a first set angle ΨS, and a second set angle θS. The SID 120 is a distance between the focus F of the radiation R and the radiation detection surface 22. The SID 120 is output from the irradiation controller 96 to the display controller 99. The SID 120 is an example of "set distance" according to the technology of the present disclosure. The first set angle ΨS and the second set angle θS are angles between the radiation generation unit 15 and the electronic cassette 11 that should be set in the imaging menus. The first set angle ΨS and the second set angle θS are set to 0° in substantially all imaging menus except for a special imaging menu. The fact that the first set angle ΨS and the second set angle θS are 0° means a state in which the radiation generation unit 15 and the electronic cassette 11 directly face each other. The first set angle ΨS and the second set angle θS are output from the irradiation controller 96 to the registration support processing unit 100 (see FIG. 16). A tube current-irradiation time product may be set as the irradiation conditions 106 instead of the tube current and the irradiation time.

The mobile radiation generation apparatus 10 receives an imaging order from the RIS through the communication unit 80. Identification data (ID) for identifying the subject H, instruction information of an imaging procedure given by a medical doctor or the like in a treatment department who issues an imaging order, and the like are registered in the imaging order. The mobile radiation generation apparatus 10 displays the imaging order received from the RIS on the display 32 according to an operation of the operator OP. The operator OP checks the contents of the imaging order through the display 32.

The mobile radiation generation apparatus 10 displays a plurality of electronic cassettes 11 stored in the cassette storage portion 30 on the display 32 such that one of the plurality of electronic cassettes 11 can be selected. The operator OP selects one electronic cassette 11 that is used to perform the imaging of the subject H indicated by the imaging order. Accordingly, the selected electronic cassette 11 and the imaging order are associated with each other.

Further, the mobile radiation generation apparatus 10 displays the imaging menus 105 on the display 32 such that the imaging menus 105 can be selected. The operator OP selects the imaging menu 105 that matches an imaging procedure designated in the imaging order. Accordingly, the imaging menu 105 is received by the receiving unit 95, and the irradiation conditions 106 corresponding to the received imaging menu 105 are read out from the irradiation condition table 91 to the receiving unit 95. Then, the irradiation conditions 106 are set in the voltage generator 48 by the irradiation controller 96. A tube voltage, a tube current, and an irradiation time of the irradiation conditions 106 read out from the irradiation condition table 91 can be finely adjusted by the operator OP via the operation panel 33 before being set in the voltage generator 48.

For example, as shown in FIG. 13, the radiation tube 47 generates the radiation R in response to the irradiation start instruction signal 108 output from the irradiation switch 34. The electronic cassette 11 performs a reset operation (not shown) for reading and discarding dark charges from the pixels of the sensor panel and then performs an accumulation operation for accumulating signal charges in the pixels, in response to the irradiation start synchronization signal 109 transmitted in conformity with the irradiation start timing for the radiation R. Further, the electronic cassette 11 performs a readout operation for reading the signal charges accumulated in the pixels and outputting the signal charges as the radiation image 24, in response to the irradiation end synchronization signal 110 transmitted in conformity with the irradiation end timing for the radiation R. A series of operations for emitting the radiation R from the radiation tube 47 in this way and outputting the radiation image 24 from the electronic cassette 11 is "radiography".

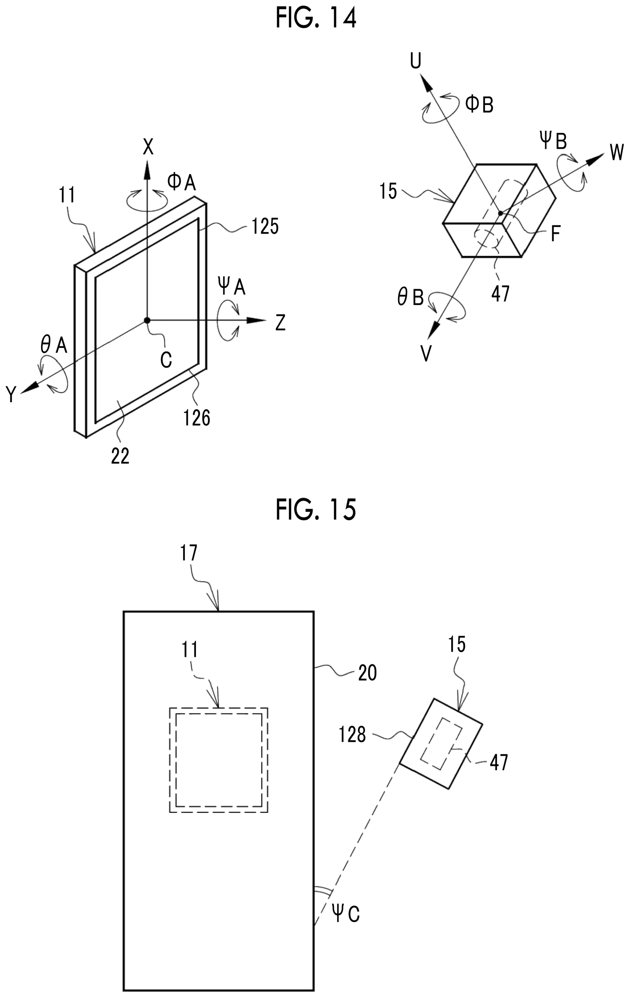

For example, as shown in FIG. 14, three-dimensional axes of an X axis, a Y axis, and a Z axis orthogonal to each other are set for the electronic cassette 11. The X axis is an axis parallel to a first side 125 of the radiation detection surface 22, and the Y axis is an axis parallel to a second side 126 of the radiation detection surface 22. In other words, the X axis is an axis parallel to an up-down direction of the radiation detection surface 22, and the Y axis is an axis parallel to a left-right direction of the radiation detection surface 22. The Z axis is a normal to the radiation detection surface 22 passing through a center C of the radiation detection surface 22. The second side 126 is an example of "a side of a radiation detection surface" according to the technology of the present disclosure.

Meanwhile, three-dimensional axes of a U axis, a V axis, and a W axis orthogonal to each other are set for the radiation generation unit 15. The U axis corresponds to the X axis and is an axis parallel to the up-down direction of the radiation generation unit 15. The V axis corresponds to the Y axis and is an axis parallel to a left-right direction of the radiation generation unit 15. The W axis corresponds to the Z axis and is an axis parallel to a front-rear direction of the radiation generation unit 15 passing through the focus F of the radiation R of the radiation tube 47. The term "orthogonal" mentioned herein refers to not only "completely orthogonal" but also "orthogonal" in the sense of including an error that is generally allowed in a technical field to which the technology of the present disclosure belongs.

The posture detection sensor 23 detects an angle θA of the electronic cassette 11 about the Y axis among an angle ΦA of the electronic cassette 11 about the X axis, an angle θA of the electronic cassette 11 about the Y axis, and an angle ΨA of the electronic cassette 11 about the Z axis. The posture detection sensor 23 outputs the detected angle θA as the posture detection result 111. The posture detection sensor 23 outputs the posture detection result 111 at a predetermined sampling rate, such as 10 times/second. The angle θA is an inclination angle of the subject H in the semi-sitting position shown in FIG. 12. The first rotational position detection sensor 64 detects an angle θB of the radiation generation unit 15 about the V axis. The first rotational position detection sensor 64 outputs the detected angle θB as the first rotational position detection result 70. The second rotational position detection sensor 67 detects an angle ΨB of the radiation generation unit 15 about the W axis. The second rotational position detection sensor 67 outputs the detected angle ΨB as the second rotational position detection result 71. The angle θA, the angle θB, and the angle ΨB take values in a range of 0° to 359°. The first rotational position detection sensor 64 and the second rotational position detection sensor 67 output the first rotational position detection result 70 and the second rotational position detection result 71 at a predetermined sampling rate, such as 10 times/second.

The registration support processing unit 100 performs processing for supporting registration between the radiation generation unit 15 and the electronic cassette 11 about the Y axis and the V axis as the registration support processing. Further, the registration support processing unit 100 performs processing for supporting registration between the radiation generation unit 15 and the electronic cassette 11 about the Z axis and the W axis as the registration support processing.

There is a problem in the registration support processing between the radiation generation unit 15 and the electronic cassette 11 about the Z axis and the W axis. This problem is that the electronic cassette 11 is not captured in the captured image 112 obtained from the camera 75 since the electronic cassette 11 is covered by the subject H as shown in FIG. 1. In a case where the electronic cassette 11 is not captured in the captured image 112, the angle ΨA of the electronic cassette 11 about the Z axis cannot be detected from the captured image 112 and registration support processing between the radiation generation unit 15 and the electronic cassette 11 about the Z axis and the W axis cannot be performed.

The electronic cassette 11 may be covered by the subject H, but the examination table 17 is not covered by the subject H. Therefore, in the technology of the present disclosure, for example, as shown in FIG. 15, an angle ΨC is detected from the captured image 112 instead of the angle ΨA. The angle ΨC is an angle about the vertical axis VA between the radiation generation unit 15 and the examination table 17. More specifically, the angle ΨC is an angle about the vertical axis VA between a side 128 of the radiation generation unit 15, which is parallel to the V axis extending to the left and right, and the long edge 20 of the examination table 17. The side 128 of the radiation generation unit 15 parallel to the V axis extending to the left and right is parallel to a side of the irradiation field of the radiation R. The angle ΨC also has a value in a range of 0° to 359° like the angle θA and the like. The angle ΨC is an example of "first angle" according to the technology of the present disclosure. Further, the long edge 20 is an example of "an edge of an examination table" according to the technology of the present disclosure. In the following description, the angle ΨC is denoted as a first angle ΨC. Using the angle ΨC instead of the angle ΨA is on the premise that the electronic cassette 11 is placed on the examination table 17 by the operator OP such that the first side 125 of the radiation detection surface 22 is parallel to the long edge 20 of the examination table 17.

For example, as shown in FIG. 16, the registration support processing unit 100 functions as a first angle detection unit 130, a second angle detection unit 131, and a first/second support information output unit 132. The captured image 112 obtained from the camera 75 is input to the first angle detection unit 130. Further, reference contour line data 133 is input to the first angle detection unit 130. The reference contour line data 133 is stored in the storage 81. The first angle detection unit 130 detects the first angle ΨC based on the captured image 112 and the reference contour line data 133. The first angle detection unit 130 outputs the first angle ΨC to the first/second support information output unit 132.

The rotational position detection result group 73 and the posture detection result 111 are input to the second angle detection unit 131. The second angle detection unit 131 detects a second angle θC based on the rotational position detection result group 73 and the posture detection result 111. The second angle θC is an angle about the Y axis between the radiation generation unit 15 and the electronic cassette 11. More specifically, the second angle θC is a difference (θC = θA - θB) between the angle θA of the electronic cassette 11 about the Y axis and the angle θB of the radiation generation unit 15 about the V axis. The second angle detection unit 131 outputs the second angle θC to the first/second support information output unit 132.

The first set angle ΨS and the second set angle θS are input to the first/second support information output unit 132. Here, the first set angle ΨS is a set angle corresponding to the first angle ΨC, and the second set angle θS is a set angle corresponding to the second angle θC. The first/second support information output unit 132 outputs a support information group 134 based on the first angle ΨC, the second angle θC, the first set angle ΨS, and the second set angle θS.

The support information group 134 includes first support information 135 and second support information 136. The first support information 135 is a first deviation amount ΔΨ between the first angle ΨC and the first set angle ΨS. Further, the second support information 136 is a second deviation amount Δθ between the second angle θC and the second set angle θS. More specifically, the first deviation amount ΔΨ is a difference (ΔΨ = ΨC - ΨS) between the first angle ΨC and the first set angle ΨS. Furthermore, the second deviation amount Δθ is a difference (Δθ = θC - θS) between the second angle θC and the second set angle θS. In a case where the first set angle ΨS and the second set angle θS are 0°, the first deviation amount ΔΨ and the second deviation amount Δθ are none other than the first angle ΨC and the second angle θC, respectively. In a case where the first deviation amount ΔΨ and the second deviation amount Δθ are in a range of 0° to 179°, the radiation generation unit 15 deviates clockwise as viewed from the electronic cassette 11 (the long edge 20 of the examination table 17 in a case of the first deviation amount ΔΨ). In a case where the first deviation amount ΔΨ and the second deviation amount Δθ are in a range of 180° to 359°, the radiation generation unit 15 deviates counterclockwise as viewed from the electronic cassette 11.

The registration support processing unit 100 detects the first angle ΨC and the second angle θC and outputs the support information group 134 each time the rotational position detection result group 73, the posture detection result 111, and the captured image 112 are input. That is, the support information group 134 is updated each time the rotational position detection result group 73, the posture detection result 111, and the captured image 112 are input.

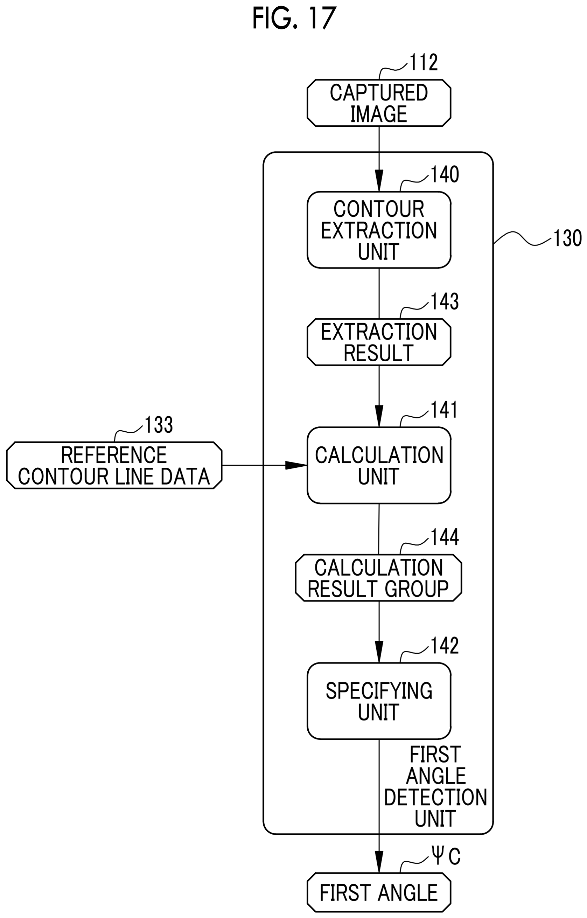

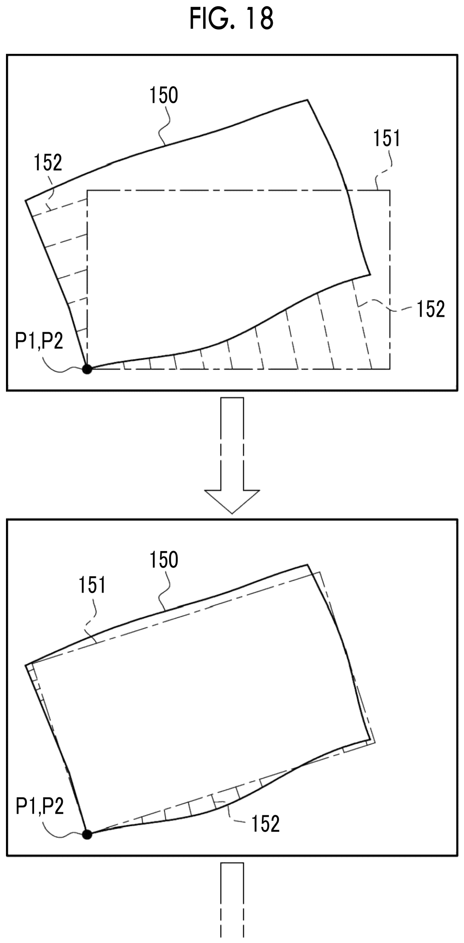

For example, as shown in FIG. 17, the first angle detection unit 130 functions as a contour extraction unit 140, a calculation unit 141, and a specifying unit 142. The captured image 112 obtained from the camera 75 is input to the contour extraction unit 140. The contour extraction unit 140 extracts a contour line 150 (see FIG. 18) of the long edges 20 and the short edges 21 of the examination table 17 from the captured image 112. The contour extraction unit 140 outputs an extraction result 143 of the contour line 150 to the calculation unit 141.

The reference contour line data 133 is input to the calculation unit 141. For example, as shown in FIG. 18, the reference contour line data 133 is data of a reference contour line 151 that is an ideal contour line in a case where both the long edges 20 and the short edges 21 of the examination table 17 are straight lines and are orthogonal to each other. The calculation unit 141 aligns any one corner point P1 of the contour line 150 of the extraction result 143 with any one corner point P2 of the reference contour line 151. Then, the calculation unit 141 sequentially calculates differences 152 between the contour line 150 and the reference contour line 151 while fixing the contour line 150 and rotating the reference contour line 151 counterclockwise, for example, by 1°. The differences 152 are distances between a plurality of set points on two orthogonal sides of the reference contour line 151 and corresponding points on two sides of the contour line 150. As the differences 152 are sequentially calculated in this way, calculation results of the differences 152 for each rotation angle of the reference contour line 151 are obtained. The calculation unit 141 outputs a calculation result group 144, which is the calculation results of the differences 152 for each rotation angle of the reference contour line 151, to the specifying unit 142.

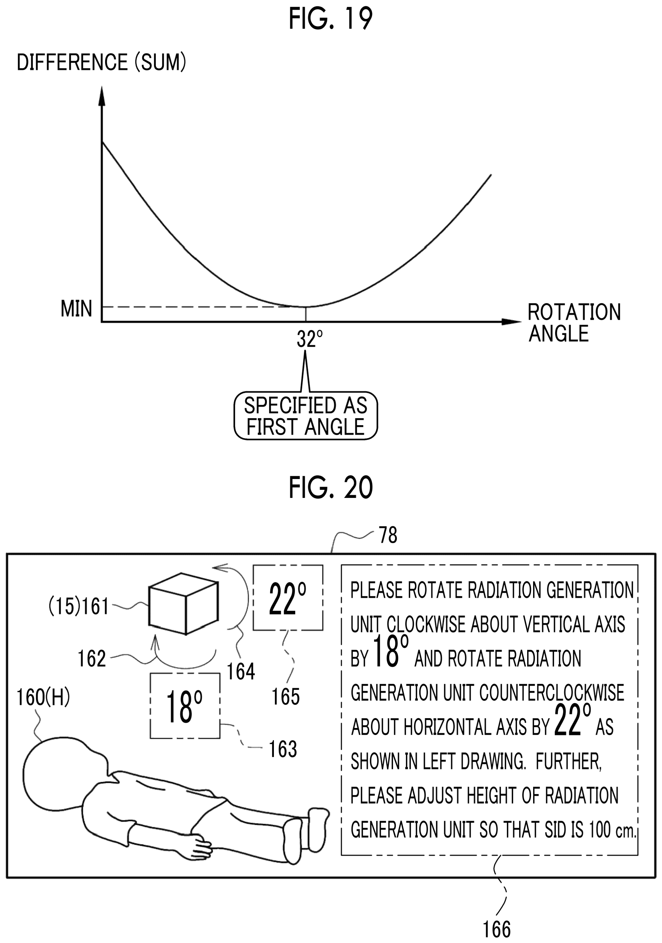

The specifying unit 142 obtains the sum of the calculation results of the plurality of differences 152 constituting the calculation result group 144. Then, for example, as shown in FIG. 19, the specifying unit 142 specifies an angle at which the sum of the differences 152 is a minimum value MIN as the first angle ΨC. The specifying unit 142 outputs the specified first angle ΨC. In a case where the sum of the previous differences 152 and the sum of the current differences 152 are compared with each other and the sum of the current differences 152 is larger than the sum of the previous differences 152, the rotation direction of the reference contour line 151 may be reversed. In this case, it is possible to quickly search for an angle at which the sum of the differences 152 is the minimum value MIN, as compared with a case where the rotation direction of the reference contour line 151 is fixed in one direction.

For example, as shown in FIG. 20, the display controller 99 causes the indicator 78 to perform a display based on the first support information 135 and a display based on the second support information 136. More specifically, an illustration 160 of the subject H and an illustration 161 of the radiation generation unit 15 are displayed on the left side in the indicator 78. Then, an arrow 162 indicating the movement direction of the radiation generation unit 15 for reducing the first deviation amount ΔΨ and a numerical value 163 of an angle representing the movement amount thereof are displayed below the illustration 161. Further, an arrow 164 indicating the movement direction of the radiation generation unit 15 for reducing the second deviation amount Δθ and a numerical value 165 of an angle representing the movement amount thereof are displayed on the right side of the illustration 161. The arrow 162 and the numerical value 163 are the display based on the first support information 135, and the arrow 164 and the numerical value 165 are the display based on the second support information 136. Furthermore, a message 166 showing the movement direction and the movement amount of the radiation generation unit 15 for reducing the first deviation amount ΔΨ and the second deviation amount Δθ is displayed on the right side in the indicator 78. The message 166 also includes a sentence relating to the adjustment of the SID 120.

The display controller 99 updates the display of the indicator 78 each time the support information group 134 is input from the registration support processing unit 100. For this reason, in a case where the operator OP moves the radiation generation unit 15 while viewing the display of the indicator 78, the display contents of the arrows 162 and 164, the numerical values 163 and 165, and the message 166 are updated.

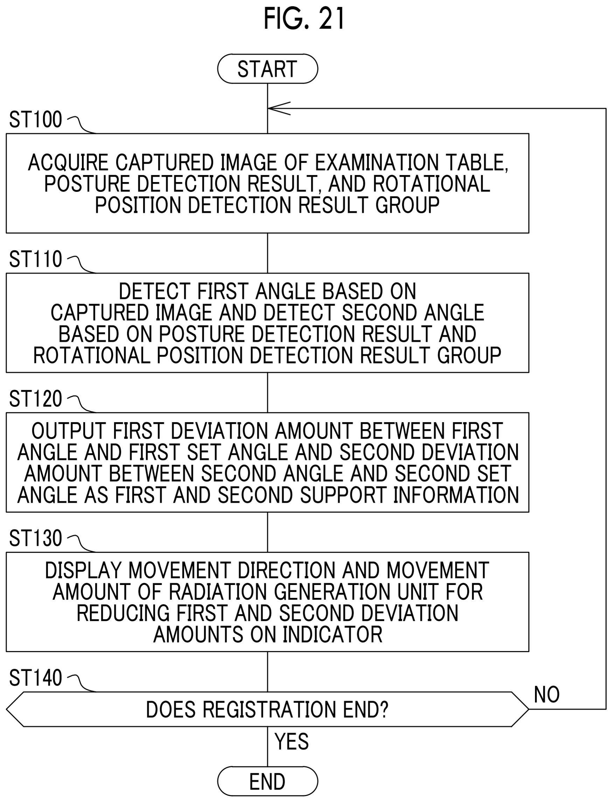

Next, the action of the above-described configuration will be described with reference to a flowchart shown in, for example, FIG. 21. As shown in FIG. 10, in a case where the operation program 90 is started, the CPU 83 functions as the receiving unit 95, the irradiation controller 96, the cassette controller 97, the display-related processing unit 98, the display controller 99, the registration support processing unit 100, and the main controller 101.

Prior to radiography, the imaging menu 105 corresponding to an imaging order is selected by the operator OP via the display 32 and the imaging menu 105 is received in the receiving unit 95. Then, the irradiation conditions 106 corresponding to the imaging menu 105 are read out from the irradiation condition table 91 by the receiving unit 95. The read-out irradiation conditions 106 are finely adjusted by the operator OP as necessary, and are then set in the voltage generator 48 by the irradiation controller 96. The SID 120 of the irradiation conditions 106 is output to the display controller 99, and the first set angle ΨS and the second set angle θS are output to the registration support processing unit 100.

Registration of the electronic cassette 11, the radiation generation unit 15, and the subject H, the adjustment of the opening degree of the emission opening of the irradiation field limiter 46, and the like are performed by the operator OP. In this case, the captured image 112 of the examination table 17 is obtained by the camera 75, and the captured image 112 is input to the registration support processing unit 100. Further, the posture detection result 111 output from the posture detection sensor 23 of the electronic cassette 11 is input to the registration support processing unit 100. Furthermore, the rotational position detection result group 73 output from the rotational position detection sensor group 72 is input to the registration support processing unit 100. That is, the captured image 112, the posture detection result 111, and the rotational position detection result group 73 are acquired by the registration support processing unit 100 (Step ST100).

The first angle ΨC is detected based on the captured image 112 by the first angle detection unit 130 of the registration support processing unit 100 (Step ST110). More specifically, as shown in FIGS. 17 to 19, the contour line 150 of the long edges 20 and the short edges 21 of the examination table 17 is extracted from the captured image 112 by the contour extraction unit 140. Next, the calculation unit 141 sequentially calculates the differences 152 between the contour line 150 and the reference contour line 151 while fixing the contour line 150 and rotating the reference contour line 151. Then, an angle at which the sum of the differences 152 is the minimum value MIN is specified as the first angle ΨC by the specifying unit 142. The first angle ΨC is output from the first angle detection unit 130 to the first/second support information output unit 132.

Further, the second angle θC is detected based on the posture detection result 111 and the rotational position detection result group 73 by the second angle detection unit 131 (Step ST110). The second angle θC is output from the second angle detection unit 131 to the first/second support information output unit 132.

As shown in FIG. 16, the first deviation amount ΔΨ between the first angle ΨC and the first set angle ΨS is output as the first support information 135 by the first/second support information output unit 132 (Step ST120). Further, the second deviation amount Δθ between the second angle θC and the second set angle θS is output as the second support information 136 (Step ST120). The support information group 134 including the first support information 135 and the second support information 136 is output from the registration support processing unit 100 (the first/second support information output unit 132) to the display controller 99.

As shown in FIG. 20, the movement direction and the movement amount of the radiation generation unit 15 for reducing the first deviation amount ΔΨ and the second deviation amount Δθ are displayed on the indicator 78 under the control of the display controller 99 (Step ST130). The operator OP moves the radiation generation unit 15 to reduce the first deviation amount ΔΨ and the second deviation amount Δθ with reference to the display of the indicator 78. More specifically, the operator OP rotates the radiation generation unit 15 about the vertical axis VA with the first rotation mechanism 60 to reduce the first deviation amount ΔΨ. Further, the operator OP rotates the radiation generation unit 15 about the horizontal axis HA with the second rotation mechanism 61 to reduce the second deviation amount Δθ.

A series of processing of Steps ST100 to ST130 is continuously repeated until the registration ends (NO in Step ST140). In a case where the registration ends (YES in Step ST140), a series of processing of Steps ST100 to ST130 also ends.

After the registration ends, the irradiation switch 34 is operated by the operator OP and the warm-up instruction signal 107 and the irradiation start instruction signal 108 are received by the receiving unit 95. Accordingly, the radiation R is emitted from the radiation tube 47 under the set irradiation conditions 106. Further, in the electronic cassette 11, the accumulation operation is performed in response to the irradiation start synchronization signal 109 and the readout operation is performed in response to the irradiation end synchronization signal 110. Accordingly, the radiation image 24 is output from the electronic cassette 11.

The radiation image 24 is received by the image receiving unit 115 of the display-related processing unit 98. The radiation image 24 is output from the image receiving unit 115 to the image processing unit 116, is subjected to various types of image processing in the image processing unit 116, and is then output to the display controller 99. Then, as shown in FIG. 3, the radiation image 24 is displayed on the display 32 under the control of the display controller 99. The radiation image 24 is transmitted to the PACS in response to an instruction from the operator OP and is stored in the PACS.

As described above, the CPU 83 includes the registration support processing unit 100. The first angle detection unit 130 of the registration support processing unit 100 detects and acquires the first angle ΨC about the vertical axis between the radiation generation unit 15 and the long edge 20 of the examination table 17. As shown in FIGS. 14 and 15, the first angle ΨC corresponds to an angle about the Z axis, which is a normal to the radiation detection surface 22 of the electronic cassette 11, between the radiation generation unit 15 and the electronic cassette 11. The first/second support information output unit 132 of the registration support processing unit 100 outputs the first support information 135 for supporting the registration of the radiation generation unit 15 with respect to an angle about the Z axis, based on the first angle ΨC. For this reason, even in a case where the electronic cassette 11 is covered by the subject H, it is possible to support registration between the radiation generation unit 15 and the electronic cassette 11.

Depending on the size of a hospital room, the disposition of the examination table 17, and the disposition of equipment, such as a locker, the mobile radiation generation apparatus 10 may have to approach the examination table 17 at an angle. In such a case, the registration of the radiation generation unit 15 with respect to an angle about the Z axis is essential. For this reason, the technology of the present disclosure is particularly suitable in a case where the mobile radiation generation apparatus 10 has to approach the examination table 17 at an angle.

As shown in FIG. 20, the display controller 99 controls the display based on the first support information 135 that is shown on the indicator 78. For this reason, the display based on the first support information 135 can be shown to the operator OP, which can support the registration of the radiation generation unit 15 to be performed by the operator OP.

As shown in FIG. 16, the first/second support information output unit 132 outputs the first deviation amount ΔΨ between the first angle ΨC and the first set angle ΨS as the first support information 135. As shown in FIG. 20, the display controller 99 performs a display, which shows the movement direction and the movement amount of the radiation generation unit 15 for reducing the first deviation amount ΔΨ, as the display based on the first support information 135. For this reason, the operator OP can reliably grasp in which direction and by what amount the radiation generation unit 15 should be moved to reduce the first deviation amount ΔΨ, which makes registration work more effective. The display based on the first support information 135 may be any of displays showing the movement direction and the movement amount. In a case where the display based on the first support information 135 is only the display showing the movement direction, it is preferable to notify the operator OP with a beep or the like when the first deviation amount ΔΨ is 0.

As shown in FIG. 16, the first angle detection unit 130 acquires the captured image 112 of the examination table 17 and detects the first angle ΨC based on the captured image 112. Accordingly, it is possible to detect the highly reliable first angle ΨC. Further, since the captured image 112 has only to capture the examination table 17, the performance of the camera 75 may not be very high. For this reason, it is possible to suppress an increase in cost required for the configuration for detecting the first angle ΨC.

As shown in FIGS. 17 to 19, the contour extraction unit 140 extracts the contour line 150 of the long edges 20 and the short edges 21 of the examination table 17 from the captured image 112. The calculation unit 141 sequentially calculates differences 152 between the contour line 150 and a pre-registered reference contour line 151 while fixing the contour line 150 and rotating the reference contour line 151. The specifying unit 142 specifies (detects) an angle at which the sum of the differences 152 is the minimum value MIN as the first angle.

For example, in a case where the examination table 17 is a bed, the edge of the examination table 17 is not necessarily straight since a sheet placed over the bed may be wavy. Therefore, the above-described processing shown in FIGS. 17 to 19 is performed to make the accuracy higher. It is possible to detect a more reliable first angle ΨC considering the realistic shape of the edge of the examination table 17. The differences 152 may be calculated while the reference contour line 151 is fixed and the contour line 150 is rotated.

As shown in FIG. 16, the second angle detection unit 131 detects and acquires the second angle θC about the Y axis, which is parallel to the second side 126 of the radiation detection surface 22, between the radiation generation unit 15 and the electronic cassette 11. The first/second support information output unit 132 outputs the second support information 136 for supporting the registration of the radiation generation unit 15 with respect to an angle about the Y axis, based on the second angle θC. For this reason, it is possible to support not only the registration of the radiation generation unit 15 with respect to an angle about the Z axis but also the registration of the radiation generation unit 15 with respect to an angle about the Y axis.

In a case where the posture of the subject H is the semi-sitting position shown in FIG. 12, the registration of the radiation generation unit 15 with respect to an angle about the Y axis is essential. For this reason, the technology of the present disclosure is particularly suitable in a case where the posture of the subject H is the semi-sitting position.

As shown in FIG. 20, the display controller 99 controls the display based on the second support information 136 that is shown on the indicator 78. For this reason, the display based on the second support information 136 can be shown to the operator OP, which can support the registration of the radiation generation unit 15 to be performed by the operator OP.

As shown in FIG. 16, the first/second support information output unit 132 outputs the second deviation amount Δθ between the second angle θC and the second set angle θS as the second support information 136. As shown in FIG. 20, the display controller 99 performs a display, which shows the movement direction and the movement amount of the radiation generation unit 15 for reducing the second deviation amount Δθ, as the display based on the second support information 136. For this reason, the operator OP can reliably grasp in which direction and by what amount the radiation generation unit 15 should be moved to reduce the second deviation amount Δθ, which makes registration work more effective. As in the case of the display based on the first support information 135, the display based on the second support information 136 may also be any of displays showing the movement direction and the movement amount. In a case where the display based on the second support information 136 is only the display showing the movement direction, it is preferable to notify the operator OP with a beep or the like when the second deviation amount Δθ is 0.

As shown in FIG. 10, the registration support processing unit 100 acquires the posture detection result 111 output from the posture detection sensor 23 provided in the electronic cassette 11. Then, as shown in FIG. 16, the second angle detection unit 131 detects the second angle θC based on the posture detection result 111. Accordingly, it is possible to detect the highly reliable second angle θC.

As shown in FIGS. 1 and 2, the mobile radiation generation apparatus 10 includes the second support column 41 and the arm part 28 attached to the second support column 41, and the radiation generation unit 15 is disposed at the distal end of the arm part 28. For this reason, the radiation generation unit 15 can be aligned with the electronic cassette 11 placed at a position away from the body part 25.

As shown in FIG. 4, the arm part 28 includes the first joint points 53A and 53B that are used to change the angle of the arm part 28 relative to the second support column 41. Accordingly, the angle of the radiation generation unit 15 can be changed.

As shown in FIG. 4, the arm part 28 includes the link mechanism 55 for keeping the radiation generation unit 15 in a horizontal posture. For this reason, the posture of the radiation generation unit 15 can be kept horizontal without troubling the operator OP.

As shown in FIG. 5, the mobile radiation generation apparatus 10 includes the first rotation mechanism 60 that rotates the radiation generation unit 15 about the vertical axis VA and the second rotation mechanism 61 that rotates the radiation generation unit 15 about the horizontal axis HA. For this reason, the radiation generation unit 15 can be rotated about the vertical axis VA and the horizontal axis HA.

As shown in FIG. 5, the first rotation mechanism 60 is positioned closer to the radiation generation unit 15 than the first joint points 53A and 53B, and the second rotation mechanism 61 is positioned closer to the radiation generation unit 15 than the first rotation mechanism 60. As compared with a case where the support column part 27 is provided with a rotation mechanism and the arm part 28 is rotated relative to the support column part 27, the arm part 28 avoids a large swing, which can reduce the risk of the mobile radiation generation apparatus 10 tipping over or the like. Further, less space is required for rotation.

Furthermore, the angle θC about the Y axis between the radiation generation unit 15 and the electronic cassette 11 has been exemplified as the second angle, but the present disclosure is not limited thereto. An angle ΦC about the X axis between the radiation generation unit 15 and the electronic cassette 11 may be detected as the second angle, instead of or in addition to the angle θC. In this case, the posture detection sensor 23 detects an angle ΦA of the electronic cassette 11 about the X axis instead of or in addition to the angle θA. In addition, a rotation mechanism that rotates the radiation generation unit 15 about a horizontal axis extending to the left and right is provided instead of or in addition to the second rotation mechanism 61.

### Second embodiment

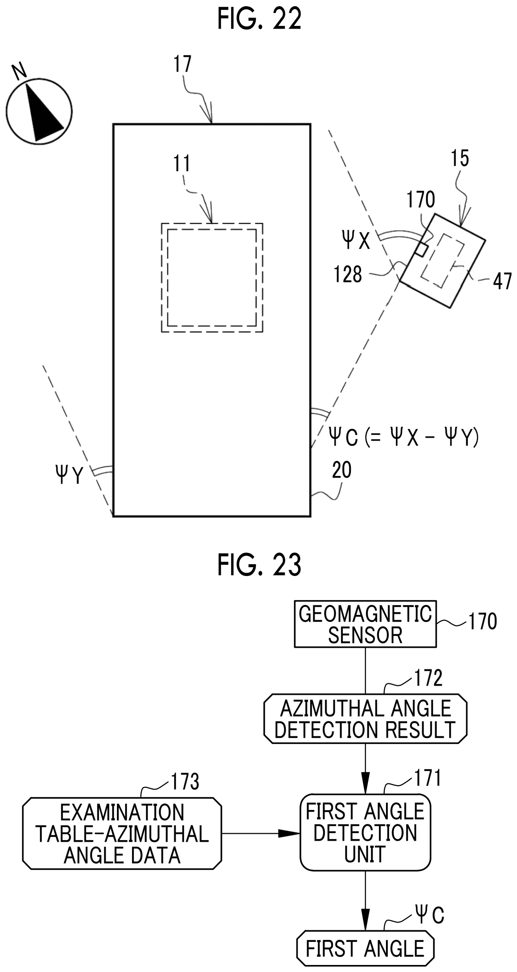

The first angle ΨC is detected based on the captured image 112 in the first embodiment, but the present disclosure is not limited thereto. For example, the radiation generation unit 15 may be provided with a geomagnetic sensor 170 as shown in FIGS. 22 and 23, and the first angle ΨC may be detected based on an output from the geomagnetic sensor 170.

In FIG. 23, an azimuthal angle detection result 172 output from the geomagnetic sensor 170 and examination table-azimuthal angle data 173 are input to a first angle detection unit 171 of the present embodiment. The azimuthal angle detection result 172 includes an angle ΨX shown in FIG. 22. The angle ΨX is an angle about the vertical axis VA between the magnetic north and the side 128 of the radiation generation unit 15 parallel to the V axis extending to the left and right. The examination table-azimuthal angle data 173 includes an angle ΨY shown in FIG. 22. The angle ΨY is an angle about the vertical axis VA between the long edge 20 of the examination table 17 and the magnetic north. The examination table-azimuthal angle data 173 is registered in the storage 81 in advance by a hospital manager or the like. The examination table-azimuthal angle data 173 is an example of "a pre-registered azimuthal angle of an examination table" according to the technology of the present disclosure.

The first angle detection unit 171 calculates a difference (ΨX - ΨY) between the angle ΨX of the azimuthal angle detection result 172 and the angle ΨY of the examination table-azimuthal angle data 173. The first angle detection unit 171 outputs the calculated difference as the first angle ΨC.

As described above, in the second embodiment, the first angle detection unit 171 acquires the azimuthal angle detection result 172 output from the geomagnetic sensor 170 and detects a difference between the azimuthal angle detection result 172 and pre-registered examination table-azimuthal angle data 173 as the first angle ΨC. For this reason, the first angle ΨC can be easily detected as compared with the first embodiment in which the first angle ΨC is detected based on the captured image 112.

### Third embodiment

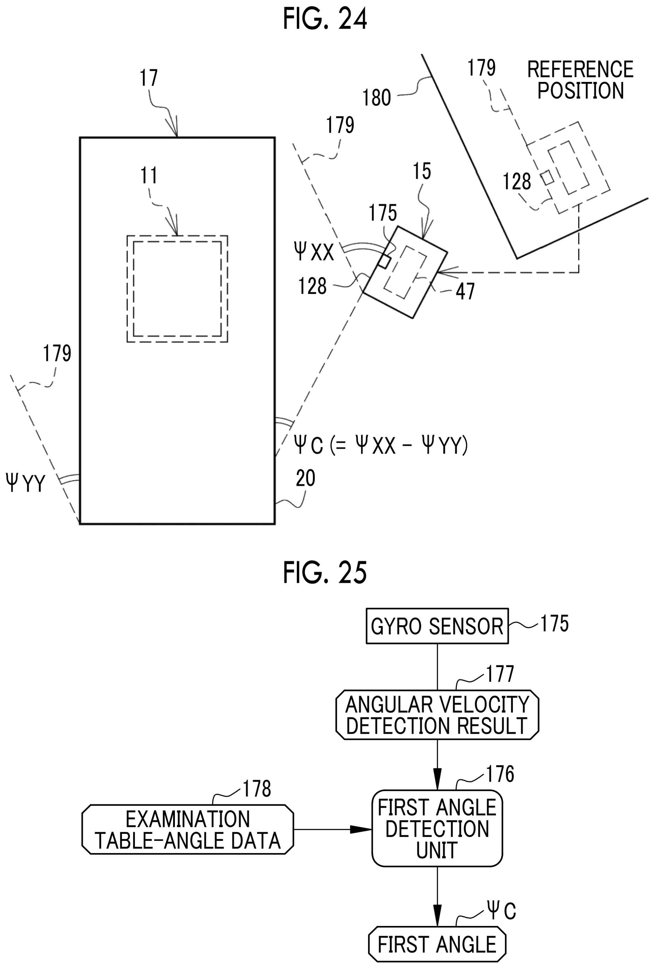

Alternatively, for example, as shown in FIGS. 24 and 25, the radiation generation unit 15 may be provided with a gyro sensor 175 and the first angle ΨC may be detected based on an output from the gyro sensor 175.

In FIG. 25, an angular velocity detection result 177 output from the gyro sensor 175 and examination table-angle data 178 are input to a first angle detection unit 176 of the present embodiment. The examination table-angle data 178 includes an angle ΨYY shown in FIG. 24. The angle ΨYY is an angle about the vertical axis VA between the long edge 20 of the examination table 17 and a reference line 179. The reference line 179 is a line that is parallel to the side 128 of the radiation generation unit 15 parallel to the V axis extending to the left and right at a preset reference position 180. The reference position 180 is, for example, a charging standby location for the mobile radiation generation apparatus 10 that is installed in the hospital. The examination table-angle data 178 is registered in the storage 81 in advance by a hospital manager or the like. The examination table-angle data 178 is an example of "a pre-registered angle of the examination table" according to the technology of the present disclosure.

The first angle detection unit 176 derives an angle ΨXX of the radiation generation unit 15 from the reference position by integrating the angular velocity of the angular velocity detection result 177. The angle ΨXX is an angle about the vertical axis VA between the side 128 of the radiation generation unit 15 parallel to the V axis extending to the left and right and the reference line 179. The first angle detection unit 176 calculates a difference (ΨXX - ΨYY) between the angle ΨXX and the angle ΨYY of the examination table-angle data 178. The first angle detection unit 176 outputs the calculated difference as the first angle ΨC.

As described above, in the third embodiment, the first angle detection unit 176 acquires the angular velocity detection result 177 output from the gyro sensor 175 and detects the difference between the angular velocity detection result 177 and the pre-registered examination table-angle data 178 as the first angle ΨC. For this reason, the first angle ΨC can be easily detected as compared with the first embodiment in which the first angle ΨC is detected based on the captured image 112.

### Fourth embodiment

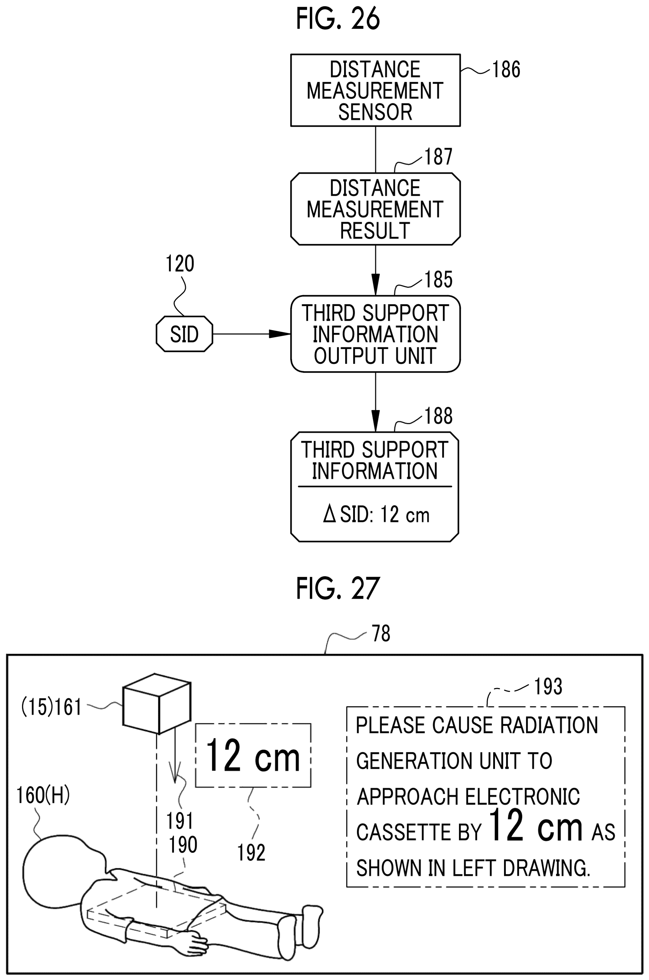

For example, as shown in FIG. 26, a registration support processing unit of a fourth embodiment functions as a third support information output unit 185 in addition to the first angle detection unit 130, the second angle detection unit 131, and the first/second support information output unit 132 of the first embodiment. A distance measurement result 187 output from a distance measurement sensor 186 and the SID 120 read out from the irradiation condition table 91 are input to the third support information output unit 185. The distance measurement sensor 186 is, for example, a stereo camera, a time of flight (ToF) camera, a light detection and ranging (LiDAR), or the like, and is provided in the radiation generation unit 15. The distance measurement sensor 186 measures a distance from between an installation location thereof and the examination table 17, and outputs the measured distance as the distance measurement result 187. The distance measurement sensor 186 outputs the distance measurement result 187 at a predetermined sampling rate, such as 10 times/second.

The third support information output unit 185 converts the distance measurement result 187 into a distance between the focus F of the radiation R and the radiation detection surface 22. In this case, the third support information output unit 185 considers a difference in height between the installation location of the distance measurement sensor 186 and the focus F, and the thickness of the electronic cassette 11. The distance measurement result 187, which is a result of measuring the distance between the installation location of the distance measurement sensor 186 and the examination table 17 as described above, corresponds to a distance between the focus F of the radiation R and the radiation detection surface 22. For this reason, the distance measurement result 187 is an example of "a distance measurement result between the focus of the radiation and the radiation detection surface" according to the technology of the present disclosure.

The third support information output unit 185 outputs a deviation amount ΔSID between the converted distance between the focus F and the radiation detection surface 22 and the SID 120 to the display controller 99 as third support information 188. The third support information output unit 185 outputs the third support information 188 each time the distance measurement result 187 is input. That is, the third support information 188 is updated each time the distance measurement result 187 is input. Incidentally, in a case where the deviation amount ΔSID has a positive value, the converted distance between the focus F and the radiation detection surface 22 is greater than the SID 120. On the other hand, in a case where the deviation amount ΔSID has a negative value, the SID 120 is greater than the converted distance between the focus F and the radiation detection surface 22.

For example, as shown in FIG. 27, the display controller 99 causes the indicator 78 to perform a display based on the third support information 188. More specifically, in the fourth embodiment, an illustration 190 of the electronic cassette 11 is displayed on the left side in the indicator 78 in addition to the illustration 160 of the subject H and the illustration 161 of the radiation generation unit 15 of the first embodiment. Further, an arrow 191 indicating the movement direction of the radiation generation unit 15 for reducing the deviation amount ΔSID and a numerical value 192 of a height representing the movement amount thereof are displayed below the illustration 161 of the radiation generation unit 15. The arrow 191 and the numerical value 192 are the display based on the third support information 188. Furthermore, a message 193 showing the movement direction and the movement amount of the radiation generation unit 15 for reducing the deviation amount ΔSID is displayed on the right side in the indicator 78.

The display controller 99 updates the display of the indicator 78 each time the third support information 188 is input from the registration support processing unit. For this reason, in a case where the operator OP moves the radiation generation unit 15 while viewing the display of the indicator 78, the display contents of the arrow 191, the numerical value 192, and the message 193 are updated.

As described above, in the fourth embodiment, the third support information output unit 185 acquires the distance measurement result 187 between the focus F of the radiation R and the radiation detection surface 22 and outputs the third support information 188 for supporting the adjustment of the distance to the SID 120 based on the distance measurement result 187. For this reason, it is possible to support the operator OP in performing the registration of the radiation generation unit 15 with respect to the SID 120. The operator OP performs the registration of the radiation generation unit 15 with respect to the SID 120 after the registration of the radiation generation unit with respect to angles about the Z axis and the Y axis ends. Specifically, the registration of the radiation generation unit 15 with respect to the SID 120 is performed by folding the arm part 28 relative to the second support column 41 at the first joint points 53A and 53B and moving the radiation generation unit 15 up and down.

The display controller 99 controls the display based on the third support information 188 that is shown on the indicator 78. For this reason, the display based on the third support information 188 can be shown to the operator OP, which can support the registration of the radiation generation unit 15 to be performed by the operator OP.

A marker having a known size may be placed on the examination table 17 to calculate a distance between the focus F of the radiation R and the radiation detection surface 22 based on the size of the marker shown in the captured image 112. Alternatively, sensors, such as rotary encoders or potentiometers, may be provided at the first joint points 53A and 53B, and the height data of the examination table 17 may be registered in advance. Then, a distance between the focus F of the radiation R and the radiation detection surface 22 may be calculated based on the outputs of the sensors and the height data.

### Fifth embodiment

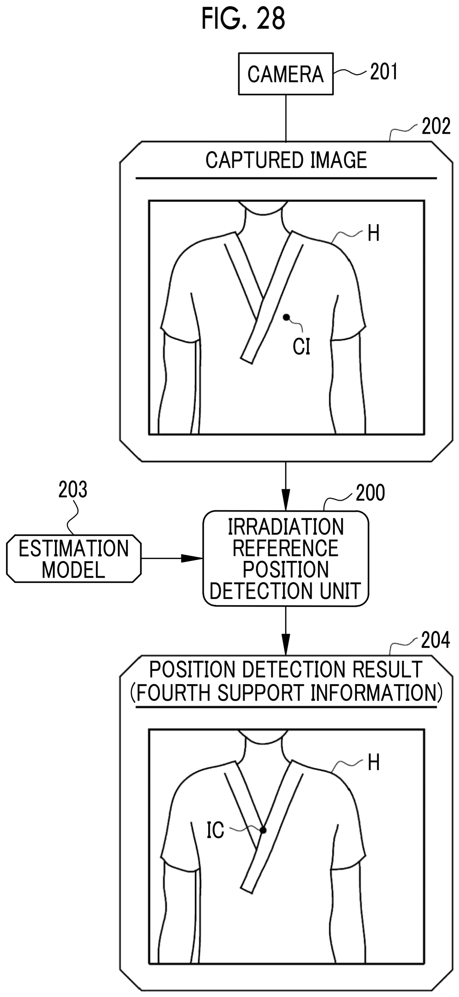

For example, as shown in FIG. 28, a registration support processing unit of a fifth embodiment functions as an irradiation reference position detection unit 200 in addition to the first angle detection unit 130, the second angle detection unit 131, and the first/second support information output unit 132 of the first embodiment. A captured image 202 of the subject H obtained from a camera 201 and an estimation model 203 are input to the irradiation reference position detection unit 200. The camera 201 is provided at a position where a center CI of the captured image 202 coincides with the focus F of the radiation R. The camera 201 images the subject H lying on the examination table 17 at a predetermined frame rate such as 10 frames/second. The estimation model 203 is a machine learning model that has been trained to output the coordinates of an irradiation reference position IC for the radiation R on the subject H in a case where the captured image 202 of the subject H is input. The irradiation reference position IC is set in advance for each imaging region. For example, in a case where the imaging region is the chest, the irradiation reference position IC is the xiphoid process.

The irradiation reference position detection unit 200 inputs the captured image 202 obtained from the camera 201 to the estimation model 203, and causes the estimation model 203 to output a position detection result 204 including coordinates of the irradiation reference position IC. The position detection result 204 is an example of "fourth support information" according to the technology of the present disclosure. The irradiation reference position detection unit 200 outputs the position detection result 204 each time the captured image 202 is input. That is, the position detection result 204 is updated each time the captured image 202 is input.

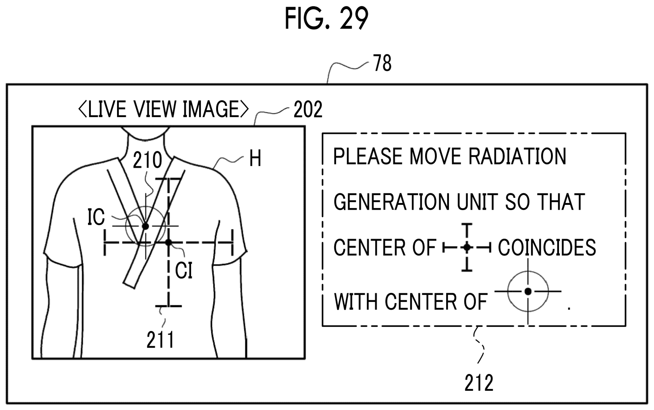

For example, as shown in FIG. 29, the display controller 99 causes the indicator 78 to perform a display based on the position detection result 204. More specifically, in the fifth embodiment, a live view image of the captured image 202 is displayed on the left side in the indicator 78. A first pointer 210 and a second pointer 211 are displayed in the captured image 202. The first pointer 210 is formed of a circle that is centered on the irradiation reference position IC and straight lines that extend vertically and horizontally from the irradiation reference position IC. The second pointer 211 is formed of a cross that is centered on the center CI of the captured image 202 coinciding with the focus F of the radiation R and four straight lines that are disposed at ends of the cross. Further, a message 212 is displayed on the right side in the indicator 78. The contents of the message 212 are to prompt the operator OP to move the radiation generation unit 15 such that the center CI of the captured image 202 which is the center of the second pointer 211, that is, the focus F of the radiation R coincides with the irradiation reference position IC which is the center of the first pointer 210.

The display controller 99 updates the display of the indicator 78 each time the position detection result 204 is input from the registration support processing unit. For this reason, in a case where the operator OP moves the radiation generation unit 15 while viewing the display of the indicator 78, the display position of the first pointer 210 is updated.

As described above, in the fifth embodiment, the irradiation reference position detection unit 200 acquires the captured image 202 of the subject H. Then, the irradiation reference position detection unit 200 detects the irradiation reference position IC for the radiation R in the subject H based on the captured image 202, and outputs the position detection result 204 as the fourth support information for supporting registration between the focus F of the radiation R and the irradiation reference position IC. For this reason, it is possible to support the operator OP in the registration of the radiation generation unit 15 with respect to the irradiation reference position IC. The operator OP performs the registration of the radiation generation unit 15 with respect to the irradiation reference position IC after the registration of the radiation generation unit with respect to angles about the Z axis and Y axis and the registration of the radiation generation unit 15 with respect to the SID 120 end. Specifically, the registration of the radiation generation unit 15 with respect to the irradiation reference position IC is performed by moving the body part 25 using the carriage unit 16.

The display controller 99 controls the display based on the position detection result 204 that is shown on the indicator 78. For this reason, the display based on the position detection result 204 can be shown to the operator OP, which can support the registration of the radiation generation unit 15 to be performed by the operator OP.

The display based on the first support information 135, the display based on the second support information 136, the display based on the third support information 188, and the display based on the position detection result 204, which is the fourth support information, may be performed on the display 32 instead of or in addition to the indicator 78. Further, projectors may be used as the first to fourth display units.

The display based on the first support information 135 may be hidden in a case where the first deviation amount ΔΨ is 0. In this case, the operator OP may be notified that the first deviation amount ΔΨ is 0 before being hidden. The same applies to the display based on the second support information 136, the display based on the third support information 188, and the display based on the position detection result 204 that is the fourth support information.

In general, the electronic cassette 11 is recognized from the captured image 112, and an angle between the first side 125 of the radiation detection surface 22 of the electronic cassette 11 and the side 128 of the radiation generation unit 15 parallel to the V axis extending to the left and right is detected as the first angle ΨC. Then, only in a case where the electronic cassette 11 cannot be recognized from the captured image 112 since the electronic cassette 11 is covered by the subject H, an angle between the side 128 of the radiation generation unit 15 parallel to the V axis extending to the left and right and the long edge 20 of the examination table 17 may be detected as the first angle ΨC. A method using a machine learning model that outputs a contour image of the electronic cassette 11 in a case where the captured image 112 in which even a part of the electronic cassette 11 is captured is input is considered as a method of recognizing the electronic cassette 11 from the captured image 112.

A mechanism that holds the radiation generation unit 15 does not necessarily need to be the illustrated link mechanism 55. The radiation generation unit 15 may be held by one arm that directly connects the radiation generation unit 15 and the support column part 27 and two joint points at which the support column part 27 and the arm are connected to each other and the arm and the radiation generation unit 15 are connected to each other. Further, the radiation generation unit 15 may be held by a telescopic mechanism that includes a support column extending in a direction of the vertical axis VA and a support column extending in a direction of the horizontal axis HA extending to the front and rear.

A motor may be added to each of the first rotation mechanism 60 and the second rotation mechanism 61 so that the first rotation mechanism 60 and the second rotation mechanism 61 are automatically rotatable without the operation of the operator OP. In this case, drive signals for driving the motors of the first rotation mechanism 60 and the second rotation mechanism 61 in a rotation direction and a rotation amount for reducing the first deviation amount ΔΨ and the second deviation amount Δθ may be output as the first support information 135 and the second support information 136.

Further, a motor may also be added to each of the first joint points 53A and 53B so that the angle of the arm part 28 relative to the support column part 27 is automatically changed without the operation of the operator OP. In this case, drive signals for driving the motors of the first joint points 53A and 53B in the direction and the amount for reducing the deviation amount ΔSID may be output as the third support information 188.

Similarly, a drive signal for moving the mobile radiation generation apparatus 10 in a direction and an amount for reducing a difference between the irradiation reference position IC and the center CI of the captured image 202 may be output as the fourth support information.

The radiation image detector is not limited to the exemplified electronic cassette 11. A computed radiography (CR) cassette may be used. Further, the subject H is not limited to the exemplified patient. The subject may be a sick animal such as a dog or cat.

In each of the above-described embodiments, for example, processing of each of the processing units, such as the receiving unit 95, the irradiation controller 96, the cassette controller 97, the display-related processing unit 98, the display controller 99, the registration support processing unit 100, the main controller 101, the image receiving unit 115, the image processing unit 116, the first angle detection unit 130, 171, and 176, the second angle detection unit 131, the first/second support information output unit 132, the contour extraction unit 140, the calculation unit 141, the specifying unit 142, the third support information output unit 185, and the irradiation reference position detection unit 200, is executed by any computer. Further, any computer may execute the processing using a processor as hardware, a program as software, or a combination thereof. In such a case, the processor is configured to execute various types of processing in each of the above-described embodiments in cooperation with the program, and may function as each unit or each means in each of the above-described embodiments. Furthermore, the execution order of the processing to be executed by the processor is not limited to the above-described order and may be changed as appropriate. Any computer may be a general-purpose computer, a computer for specific use, a workstation, or another system that can execute each processing.

The processor may be formed of one or a plurality of types of hardware, and the type of the hardware is not limited. For example, the processor may be configured with hardware such as the exemplified CPU 83, a micro processing unit (MPU), a programmable logic device such as a field programmable gate array (FPGA), a dedicated circuit for executing specific processing such as an application specific integrated circuit (ASIC), a graphic processing unit (GPU), or a neural processing unit (NPU). Further, the types of hardware may be a combination of different types of hardware. In a case where a plurality of types of hardware are configured to execute one or a plurality of types of processing of a certain processor, the plurality of types of hardware may be present in devices physically separated from each other or may be present in the same device. Furthermore, in any of the embodiments, the order of each processing executed by the processor is not limited to the above-described order, and may be changed as appropriate. The hardware is configured with an electric circuit (circuitry) in which circuit elements such as semiconductor elements are combined.

In addition, the program may be software such as firmware or a microcode. Further, the program may be, for example, a program module group, and each function thereof may be implemented by a processor configured to execute each function. The program may be a program code or a plurality of code segments stored in one or a plurality of non-transitory computer-readable media (for example, storage media or other storages). The program may be divided and stored in a plurality of non-transitory computer-readable media present in devices physically separated from each other. The program code or the code segment may represent any combination of procedures, functions, subprograms, routines, subroutines, modules, software packages, classes, instructions, data structures, or program statements. The program code or the code segment may be connected to another code segment or a hardware circuit by transmitting and receiving information, data, arguments, parameters, or contents of the memory.

It is possible to understand the technologies described in following supplementary claims from the above description.

### Supplementary claim 1

A mobile radiation generation apparatus that includes a radiation generation unit emitting radiation to a radiation image detector disposed between a subject and an examination table on which the subject lies, comprising:
a processor,
in which the processor is configured to:
   acquire a first angle about a vertical axis between the radiation generation unit and an edge of the examination table that corresponds to an angle about a normal to a radiation detection surface of the radiation image detector between the radiation generation unit and the radiation image detector; and
   output first support information for supporting registration of the radiation generation unit with respect to the angle about the normal based on the first angle.

### Supplementary claim 2

The mobile radiation generation apparatus according to supplementary claim 1, further comprising:
a first display unit,
in which the processor is configured to control a display based on the first support information that is shown on the first display unit.

### Supplementary claim 3

The mobile radiation generation apparatus according to supplementary claim 2,
in which the processor is configured to:
output a first deviation amount between the first angle and a first set angle as the first support information; and
perform a display, which shows a movement direction and/or a movement amount of the radiation generation unit for reducing the first deviation amount, as the display based on the first support information.

### Supplementary claim 4

The mobile radiation generation apparatus according to any one of supplementary claims 1 to 3,
in which the processor is configured to:
acquire a captured image of the examination table; and
detect the first angle based on the captured image.

### Supplementary claim 5

The mobile radiation generation apparatus according to supplementary claim 4,
in which the processor is configured to:
extract a contour line of the edge of the examination table from the captured image, and
detect an angle at which a difference between the contour line and a pre-registered reference contour line is a minimum value, as the first angle.

### Supplementary claim 6

The mobile radiation generation apparatus according to any one of supplementary claims 1 to 3, further comprising:
a geomagnetic sensor,
in which the processor is configured to:
   acquire an azimuthal angle detection result output from the geomagnetic sensor; and
   detect a difference between the azimuthal angle detection result and a pre-registered azimuthal angle of the examination table as the first angle.

### Supplementary claim 7

The mobile radiation generation apparatus according to any one of supplementary claims 1 to 3, further comprising:
a gyro sensor,
in which the processor is configured to:
   acquire an angular velocity detection result output from the gyro sensor; and
   detect a difference between the angular velocity detection result and a pre-registered angle of the examination table as the first angle.

### Supplementary claim 8

The mobile radiation generation apparatus according to any one of supplementary claims 1 to 7,
in which the processor is configured to:
acquire even a second angle about an axis, which is parallel to a side of the radiation detection surface, between the radiation generation unit and the radiation image detector; and
output second support information for supporting registration of the radiation generation unit with respect to an angle about the axis based on the second angle.

### Supplementary claim 9

The mobile radiation generation apparatus according to supplementary claim 8, further comprising:
a second display unit,
in which the processor is configured to control a display based on the second support information that is shown on the second display unit.

### Supplementary claim 10

The mobile radiation generation apparatus according to supplementary claim 9,
in which the processor is configured to:
output a second deviation amount between the second angle and a second set angle as the second support information; and
perform a display, which shows a movement direction and/or a movement amount of the radiation generation unit for reducing the second deviation amount, as the display based on the second support information.

### Supplementary claim 11

The mobile radiation generation apparatus according to any one of supplementary claims 8 to 10,
in which the processor is configured to:
acquire a posture detection result output from a posture detection sensor provided in the radiation image detector; and
detect the second angle based on the posture detection result.

### Supplementary claim 12

The mobile radiation generation apparatus according to any one of supplementary claims 1 to 11, further comprising:
a support column; and
an arm part that is attached to the support column,
in which the radiation generation unit is disposed at a distal end of the arm part.

### Supplementary claim 13

The mobile radiation generation apparatus according to supplementary claim 12,
in which the arm part includes a joint point that is used to change an angle of the arm part relative to the support column.

### Supplementary claim 14

The mobile radiation generation apparatus according to supplementary claim 12 or 13,
in which the arm part includes a link mechanism for keeping the radiation generation unit in a horizontal posture.

### Supplementary claim 15

The mobile radiation generation apparatus according to any one of supplementary claims 1 to 14, further comprising:
a first rotation mechanism that rotates the radiation generation unit about the vertical axis; and
a second rotation mechanism that rotates the radiation generation unit about a horizontal axis.

### Supplementary claim 16

The mobile radiation generation apparatus according to supplementary claim 15, further comprising:
a support column; and
an arm part that is attached to the support column,
in which the radiation generation unit is disposed at a distal end of the arm part,
the arm part is connected to a joint point that is used to change an angle of the arm part relative to the support column,
the first rotation mechanism is positioned closer to the radiation generation unit than the joint point, and
the second rotation mechanism is positioned closer to the radiation generation unit than the first rotation mechanism.

### Supplementary claim 17

The mobile radiation generation apparatus according to any one of supplementary claims 1 to 16,
in which the processor is configured to:
acquire a distance measurement result between a focus of the radiation and the radiation detection surface; and
output third support information for supporting adjustment of a distance between the focus of the radiation and the radiation detection surface to a set distance based on the distance measurement result.

### Supplementary claim 18

The mobile radiation generation apparatus according to supplementary claim 17, further comprising:
a third display unit,
in which the processor is configured to control a display based on the third support information that is shown on the third display unit.

### Supplementary claim 19

The mobile radiation generation apparatus according to any one of supplementary claims 1 to 18,
in which the processor is configured to:
acquire a captured image of the subject;
detect an irradiation reference position for the radiation on the subject based on the captured image; and
output fourth support information for supporting registration between a focus of the radiation and the irradiation reference position.

### Supplementary claim 20

The mobile radiation generation apparatus according to supplementary claim 19, further comprising:
a fourth display unit,
in which the processor is configured to control a display based on the fourth support information that is shown on the fourth display unit.

In the technology of the present disclosure, the above-described various embodiments and/or various modification examples may be combined with each other as appropriate. Further, it goes without saying that the present disclosure is not limited to each of the embodiments described above, various configurations can be adopted as long as the configuration does not deviate from the gist. Furthermore, the technology of the present disclosure extends to a storage medium that non-transitorily stores the program, and a computer program product including the program, in addition to the program.

The above descriptions and illustrations are detailed descriptions of portions related to the technology of the present disclosure and are merely examples of the technology of the present disclosure. For example, the description of the configuration, functions, actions, and effects having been described above is the description of examples of the configuration, functions, actions, and effects of the portions according to the technology of the present disclosure. Accordingly, it goes without saying that unnecessary portions may be deleted or new elements may be added or replaced in the description contents and shown contents described above without departing from the scope of the technology of the present disclosure. Further, in order to avoid complications and facilitate understanding of the parts related to the technology of the present disclosure, descriptions of common general knowledge and the like that do not require special descriptions for enabling the implementation of the technology of the present disclosure are omitted, in the contents described and shown above.

In the present specification, the term "A and/or B" is synonymous with the term "at least one of A or B". That is, "A and/or B" may mean only A, may mean only B, or may mean a combination of A and B. In addition, in the present specification, the same approach as "A and/or B" is applied to a case in which three or more matters are represented by connecting the matters with "and/or".

All documents, patent applications, and technical standards described in this specification are built into this specification by reference to the same extent as in a case where each of the documents, the patent applications, and the technical standards are specifically and individually indicated to be incorporated by reference.

### Explanation of References

2: radiography system
10: mobile radiation generation apparatus
11: electronic cassette
15: radiation generation unit
16: carriage unit
17: examination table
18: front wheel
19: rear wheel
20: long edge
21: short edge
22: radiation detection surface
23: posture detection sensor
24: radiation image
25: body part
26: central part
27: support column part
28: arm part
29: UI-based device
30: cassette storage portion
31: handle
32: touch panel display (display)
33: operation panel
34: irradiation switch
40: first support column
41: second support column
45: radiation source
46: irradiation field limiter
47: radiation tube
48: voltage generator
50A, 50B: arm
51: first connecting member
52: second connecting member
53A, 53B: first joint point
54A, 54B: second joint point
55: link mechanism
60: first rotation mechanism
61: second rotation mechanism
62: first rotating shaft
63: first bearing
64: first rotational position detection sensor
65: second rotating shaft
66: second bearing
67: second rotational position detection sensor
70: first rotational position detection result
71: second rotational position detection result
72: rotational position detection sensor group
73: rotational position detection result group
75, 201: camera
78: indicator
80: communication unit
81: storage
82: memory
83: CPU
84: busline
85: power feed unit
86: battery
90: operation program
91: irradiation condition table
95: receiving unit
96: irradiation controller
97: cassette controller
98: display-related processing unit
99: display controller
100: registration support processing unit
101: main controller
105: imaging menu
106: irradiation condition
107: warm-up instruction signal
108: irradiation start instruction signal
109: irradiation start synchronization signal
110: irradiation end synchronization signal
111: posture detection result
112, 202: captured image
115: image receiving unit
116: image processing unit
120: SID
125: first side of radiation detection surface
126: second side of radiation detection surface
128: side of radiation generation unit parallel to V axis extending to left and right
130, 171, 176: first angle detection unit
131: second angle detection unit
132: first/second support information output unit
133: reference contour line data
134: support information group
135: first support information
136: second support information
140: contour extraction unit
141: calculation unit
142: specifying unit
143: extraction result
144: calculation result group
150: contour line
151: reference contour line
152: difference
160: illustration of subject
161: illustration of radiation generation unit
162, 164, 191: arrow
163, 165, 192: numerical value
166, 193, 212: message
170: geomagnetic sensor
172: azimuthal angle detection result
173: examination table-azimuthal angle data
175: gyro sensor
177: angular velocity detection result
178: examination table-angle data
179: reference line
180: reference position
185: third support information output unit
186: distance measurement sensor
187: distance measurement result
188: third support information
190: illustration of electronic cassette
200: irradiation reference position detection unit
203: estimation model
204: position detection result
210: first pointer
211: second pointer
Δθ: second deviation amount
ΔΨ: first deviation amount
ΔSID: deviation amount
θA: angle of electronic cassette about Y axis
ΦA: angle of electronic cassette about X axis
ΨA: angle of electronic cassette about Z axis
θB: angle of radiation generation unit about V axis
ΦB: angle of radiation generation unit about U axis
ΨB: angle of radiation generation unit about W axis
θC: angle (second angle) about Y axis between radiation generation unit and electronic cassette
ΦC: angle (second angle) about X axis between radiation generation unit and electronic cassette
ΨC: angle (first angle) about Z axis between radiation generation unit and electronic cassette
θS: second set angle
ΨS: first set angle
ΨX: angle between side of radiation generation unit parallel to V axis extending to left and right and magnetic north
ΨXX: angle between side of radiation generation unit parallel to V axis extending to left and right and reference line
ΨY: angle between long edge of examination table and magnetic north
ΨYY: angle between long edge of examination table and reference line
C: center of radiation detection surface
CA1: central axis of first rotating shaft
CA2: central axis of second rotating shaft
CI: center of captured image
F: focus
H: subject
HA: horizontal axis
IC: irradiation reference position
MIN: minimum value of sum of differences
OP: operator
P1: any corner point of contour line
P2: any corner point of reference contour line
R: radiation
ST100, ST110, ST120, ST130, ST140: step
VA: vertical axis

## Claims

1. A mobile radiation generation apparatus that includes a radiation generation unit configured for emitting radiation to a radiation image detector disposed between a subject and an examination table on which the subject lies, comprising:
a processor,
wherein the processor is configured to:
acquire a first angle about a vertical axis between the radiation generation unit and an edge of the examination table that corresponds to an angle about a normal to a radiation detection surface of the radiation image detector between the radiation generation unit and the radiation image detector; and
output first support information for supporting registration of the radiation generation unit with respect to the angle about the normal based on the first angle.

2. The mobile radiation generation apparatus according to claim 1, further comprising:
a first display unit,
wherein the processor is configured to control a display based on the first support information that is shown on the first display unit.

3. The mobile radiation generation apparatus according to claim 2,
wherein the processor is configured to:
output a first deviation amount between the first angle and a first set angle as the first support information; and
perform a display, which shows a movement direction and/or a movement amount of the radiation generation unit for reducing the first deviation amount, as the display based on the first support information.

4. The mobile radiation generation apparatus according to any one of claims 1 to 3,
wherein the processor is configured to:
acquire a captured image of the examination table; and
detect the first angle based on the captured image.

5. The mobile radiation generation apparatus according to claim 4,
wherein the processor is configured to:
extract a contour line of the edge of the examination table from the captured image, and
detect an angle at which a difference between the contour line and a pre-registered reference contour line is a minimum value, as the first angle.

6. The mobile radiation generation apparatus according to any one of claims 1 to 3, further comprising:
a geomagnetic sensor,
wherein the processor is configured to:
acquire an azimuthal angle detection result output from the geomagnetic sensor; and
detect a difference between the azimuthal angle detection result and a pre-registered azimuthal angle of the examination table as the first angle.

7. The mobile radiation generation apparatus according to any one of claims 1 to 3, further comprising:
a gyro sensor,
wherein the processor is configured to:
acquire an angular velocity detection result output from the gyro sensor; and
detect a difference between the angular velocity detection result and a pre-registered angle of the examination table as the first angle.

8. The mobile radiation generation apparatus according to any one of claims 1 to 7,
wherein the processor is configured to:
acquire even a second angle about an axis, which is parallel to a side of the radiation detection surface, between the radiation generation unit and the radiation image detector; and
output second support information for supporting registration of the radiation generation unit with respect to an angle about the axis based on the second angle.

9. The mobile radiation generation apparatus according to claim 8, further comprising:
a second display unit,
wherein the processor is configured to control a display based on the second support information that is shown on the second display unit.

10. The mobile radiation generation apparatus according to claim 9,
wherein the processor is configured to:
output a second deviation amount between the second angle and a second set angle as the second support information; and
perform a display, which shows a movement direction and/or a movement amount of the radiation generation unit for reducing the second deviation amount, as the display based on the second support information.

11. The mobile radiation generation apparatus according to any one of claims 8 to 10,
wherein the processor is configured to:
acquire a posture detection result output from a posture detection sensor provided in the radiation image detector; and
detect the second angle based on the posture detection result.

12. The mobile radiation generation apparatus according to any one of claims 1 to 11, further comprising:
a support column; and
an arm part that is attached to the support column,
wherein the radiation generation unit is disposed at a distal end of the arm part.

13. The mobile radiation generation apparatus according to claim 12 or 13,
wherein the arm part includes a joint point that is used to change an angle of the arm part relative to the support column.

14. The mobile radiation generation apparatus according to claim 12,
wherein the arm part includes a link mechanism for keeping the radiation generation unit in a horizontal posture.

15. The mobile radiation generation apparatus according to any one of claims 1 to 14, further comprising:
a first rotation mechanism that rotates the radiation generation unit about the vertical axis; and
a second rotation mechanism that rotates the radiation generation unit about a horizontal axis.

16. The mobile radiation generation apparatus according to claim 15, further comprising:
a support column; and
an arm part that is attached to the support column,
wherein the radiation generation unit is disposed at a distal end of the arm part,
the arm part is connected to a joint point that is used to change an angle of the arm part relative to the support column,
the first rotation mechanism is positioned closer to the radiation generation unit than the joint point, and
the second rotation mechanism is positioned closer to the radiation generation unit than the first rotation mechanism.

17. The mobile radiation generation apparatus according to any one of claims 1 to 16,
wherein the processor is configured to:
acquire a distance measurement result between a focus of the radiation and the radiation detection surface; and
output third support information for supporting adjustment of a distance between the focus of the radiation and the radiation detection surface to a set distance based on the distance measurement result.

18. The mobile radiation generation apparatus according to claim 17, further comprising:
a third display unit,
wherein the processor is configured to control a display based on the third support information that is shown on the third display unit.

19. The mobile radiation generation apparatus according to any one of claims 1 to 18,
wherein the processor is configured to:
acquire a captured image of the subject;
detect an irradiation reference position for the radiation on the subject based on the captured image; and
output fourth support information for supporting registration between a focus of the radiation and the irradiation reference position.

20. The mobile radiation generation apparatus according to claim 19, further comprising:
a fourth display unit,
wherein the processor is configured to control a display based on the fourth support information that is shown on the fourth display unit.

21. A method of operating a mobile radiation generation apparatus that includes a radiation generation unit configured for emitting radiation to a radiation image detector disposed between a subject and an examination table on which the subject lies, the method comprising:
acquiring a first angle about a vertical axis between the radiation generation unit and an edge of the examination table that corresponds to an angle about a normal to a radiation detection surface of the radiation image detector between the radiation generation unit and the radiation image detector; and
outputting first support information for supporting registration of the radiation generation unit with respect to the angle about the normal based on the first angle.

22. A computer-readable storage medium storing an operation program for a mobile radiation generation apparatus that includes a radiation generation unit configured for emitting radiation to a radiation image detector disposed between a subject and an examination table on which the subject lies, the operation program configured to cause a computer to execute processing comprising:
acquiring a first angle about a vertical axis between the radiation generation unit and an edge of the examination table that corresponds to an angle about a normal to a radiation detection surface of the radiation image detector between the radiation generation unit and the radiation image detector; and
outputting first support information for supporting registration of the radiation generation unit with respect to the angle about the normal based on the first angle.
